# EUROPEAN PATENT SPECIFICATION

(11) **EP 1 915 967 B1**
(45) Date of publication and mention of the grant of the patent: **10.03.2010**
(21) Application number: 07119215.7
(22) Date of filing: 24.10.2007
(51) Int. Cl.: A61B 19/00

(54) **Manipulator system**
Manipulatorsystem
Système manipulateur

(30) Priority: 25.10.2006 US 862823 P; 08.08.2007 JP 2007206850
(43) Date of publication of application: 30.04.2008
(60) Divisional application: 09150318.5
(73) Proprietor: Terumo Kabushiki Kaisha, Tokyo 151-0072 (JP); Kabushiki Kaisha Toshiba, Minato-ku, Tokyo 105-8001 (JP)
(72) Inventor: Omori, Shigeru, Ashigarakami-gun Kanagawa 259-0151 (JP); Sano, Hiroaki, Fujinomiya-shi Shizuoka 418-0015 (JP); Hitotsuyanagi, Masao, Shibuya-ku Tokyo 151-0072 (JP); Jinno, Makoto, Minato-ku, Tokyo 105-8001 (JP)
(74) Representative: TBK-Patent

(56) References cited:
- EP-A- 1 366 776
- WO-A-00/33755
- WO-A-01/08591
- WO-A-96/27845
- US-A1- 2004 133 189
- US-A1- 2006 149 126

## Description

### BACKGROUND OF THE INVENTION

### Field of the Invention:

The present invention relates to a manipulator and a manipulator system for controlling the manipulator, more specifically to a manipulator system comprising a manipulator, which contains an actuator unit having an actuator and a working unit, attachable to and detachable from the actuator unit, having a working portion movable in conjunction with the actuator.

### Description of the Related Art:

According to laparoscopic surgery, it is customary to form a plurality of holes in the abdominal part of the patient, insert an endoscope and a manipulator (or forceps) into the respective holes, and perform the surgical operation while images captured by the endoscope are being observed on a display monitor by the surgeon. Since such a laparoscopic surgical operation does not require the abdominal cavity to be opened, the burden on the patient is small and the number of days which the patient needs to recover and spend in the hospital until they are allowed to come out of hospital is greatly reduced. For these reasons, the laparoscopic surgical operation is expected to find an increased range of applications.

A manipulator system is composed of a manipulator body and a controller therefor, as described in Japanese Laid-Open Patent Publication No. 2004-105451, for example. The manipulator body contains an operating unit controlled by human and a working unit interchangeably removable from the operating unit.

The working unit has a slender connecting shaft and an end working portion (also referred to as an end effector) disposed at the distal end of the connecting shaft. A motor for driving the end working portion via a wire is disposed in the operating unit. The wire is wound around a pulley in the vicinity of the proximal end. The motor in the operating unit is driven by the controller, whereby the wire is moved via the pulley.

The working unit does not contain an electric device such as a sensor in view of easily carrying out washing and sterilization. The positions or original points of the end working portion and the proximal end pulley cannot be directly detected, and the position of the end working portion is calculated based on rotation of the motor.

The working unit may be a gripper, a pair of scissors, an electric surgical knife, an ultrasonic surgical knife, a medical drill, or the like, and may be selected depending on a procedure in a laparoscopic operation. The working unit is removable from the operating unit, and when the working unit is attached to the operating unit, the pulley at the proximal end is engaged with a rotary shaft of the motor in the operating unit.

In such a system intended to connect a plurality of different working units to one operating unit, it is necessary to determine a motor phase as only one position, at which all the working units can be attached and detached (see Japanese Laid-Open Patent Publication No. 2004-105451, for example). The position is referred to as the original point (or the initial position).

Conventional manipulator systems are described in Japanese Laid-Open Patent Publication Nos. 2004-105451 and 2004-208922, US. Patent No. 6331181, for example.

In a system proposed in Japanese Laid-Open Patent Publication No. 2004-105451, it is unnecessary to take into consideration the motor excitation switching and electrical structure when a working unit is attached or detached.

In a system described in Japanese Laid-Open Patent Publication No. 2004-208922, a plurality of end tools (working units) are electrically attached and detached.

In a system described in US. Patent No. 6331181, a memory for obtaining identification data is mounted in a medical manipulator disposed at a distal end, and a controller receives the identification data to control a tool. The memory is an ROM, a flash memory, or the like, and the identification data is transmitted through an electrical contact.

When surgery is traditionally performed, there is a long incision made so the surgeon can view and repair the internal parts of the patient. The long incision site can be a significant concern because it is subject to infection and is often the most traumatic and painful part of the patient's recover. In recent years, many surgeons have been using endoscopic tools and performing minimally invasive surgery, thereby vastly reducing the size of the incision.

Robotic tools have been developed to further improve the minimally invasive surgical process. These tools are highly specialized. They must perform the function that a surgeon would in a miniaturized manner. Surgeons perform many different functions on internal organs such as cutting, scraping, and suturing. Different surgical tools are required for each of these functions. A different surgical device could be made for each surgical tool, but it is more cost effective to simply change the surgical tool mounted to the surgical device for each function. To control the surgical tool correctly, the robotic surgical device must identify the surgical tool attached to the surgical instrument.

In a case where various working units are attached to an actuator unit, it is necessary for a controller to correctly control each working unit in accordance with the type, and accordingly the identification data thereof is required.

Further, the position of the end working portion is calculated based on, e.g. the original point of the motor as described above. Thus, when a working unit is changed with another working unit during an operation, it is desirable that the working unit is detached from the actuator unit after precisely placed at an axial position corresponding to the original point. When the working unit is detached in a state where it is not placed at the position corresponding to the original point, a certain alarm may be sounded for the working unit to be reattached. In this case, the working unit needs to be identified to prevent the attachment of another working unit.

In the above system of US. Patent No. 6331181, the identification data of the tool can be read from the ROM, and the working unit can be controlled based on the identification data in accordance with the type. However, as described above, it is desirable that the working unit contains no electron devices, particularly no electrical contacts, in view of easily carrying out washing and sterilization after an operation.

It is preferred that the usage history of each working unit can be obtained based on the identification data from the viewpoint of easily managing the working unit. However, in a case where a plurality of controllers to be connected are provided, when the usage history of a working unit is stored in a certain controller and then the working unit is connected to another controller, the other controller inconveniently cannot recognize the usage history of the working unit.

The US 2004/133189 A1 discloses a surgical operation apparatus comprising a hook type treatment jig provided with a probe. The hook type treatment jig contains an energy receiving unit connector which has an information exchange unit and a storage unit storing ID information of a surgical instrument. An energy transmitting connector of the surgical operation apparatus also has an information exchange unit. When the energy transmission unit connector is coupled to the energy receiving unit connector, the information exchange unit reads information peculiar to the hook type treatment jig from the identification information storage unit by radio. A control circuit recognizes a type of a treatment jig as, for example, a hook type treatment jig based on information read by the information exchange unit to set a drive condition in an oscillation circuit in a state suited to driving of the probe.

### SUMMARY OF THE INVENTION

It is an object of the present invention to provide a manipulator system which is capable of obtaining identification data of a working unit by using a radio wave under reduced electric power, at the same time avoiding interference, in which the working unit can be easily washed and sterilized and has a structure without electrical contacts in a manipulator.

According to the present invention, this object is achieved by a manipulator system of claim 1.

The manipulator system comprises a manipulator and a controller therefor, and the manipulator comprises an actuator unit and a working unit attachable to and detachable from the actuator unit. The actuator unit contains an actuator, the working unit contains a shaft and an end working portion disposed at a distal end of the shaft, and the end working portion is rotated in conjunction with the actuator around an axis non-parallel to the shaft. The working unit further contains an ID holder for holding an identification signal, and the actuator unit further contains an ID recognizer for recognizing the identification signal in the ID holder and supplying the identification signal to the controller, the ID recognizer being not in contact with the ID holder. The controller controls the working unit based on the supplied identification signal.

Thus, the ID holder and the ID recognizer, which are not in contact with each other, are used for obtaining the identification data of the working unit, so that the working unit can be free of electrical contacts. Therefore, the working unit can be easily washed and sterilized, and a communication error is not caused owing to the absence of electrical contacts in the system. Electrically noncontact type devices are generally more durable than contact type devices.

An exemplary embodiment of the present invention provides a device and a method of identifying the surgical tool mounted to a robotic surgical device so that the robotic control system can correctly and reliably control the surgical tool during a surgical procedure. In an exemplary embodiment, the device includes, but is not limited to, a surgical instrument and a surgical tool control mechanism. The surgical instrument includes, but is not limited to, a surgical tool and a transmitter capable of transmitting an identifier that identifies the surgical tool. The surgical tool control mechanism includes, but is not limited to, a receiver capable of receiving the transmitted identifier.

In another exemplary embodiment, the device includes, but is not limited to, a surgical instrument and a surgical tool control mechanism. The surgical instrument includes, but is not limited to, a surgical tool and an identifier that identifies the surgical tool. The surgical tool control mechanism includes, but is not limited to, an identification reader capable of reading the identifier.

Yet another exemplary embodiment provides a method of automatically identifying a surgical tool for use during minimally invasive surgery. The method includes, but is not limited to, receiving an identifier at a receiver and controlling a surgical tool based on the received identifier. The identifier identifies the surgical tool mounted on a surgical instrument.

Yet another exemplary embodiment provides a method of automatically identifying a surgical tool for use during minimally invasive surgery. The method includes, but is not limited to, reading an identifier on a surgical instrument and controlling a surgical tool based on the read identifier. The identifier identifies the surgical tool mounted on a surgical instrument.

The above and other objects, features and advantages of the aspects will become more apparent from the following description when taken in conjunction with the accompanying drawings in which a preferred embodiment of the present invention is shown by way of illustrative example.

### BRIEF DESCRIPTION OF THE DRAWINGS

FIG. 1 is a schematic view of a manipulator system according to an unclaimed example;
FIG. 2 is an explanatory view of combination of components for the manipulator system according to the unclaimed example;
FIG. 3 is a side view of the manipulator, a working unit being separated from an operating unit;
FIG. 4 is a perspective view of the operating unit;
FIG. 5 is a partially sectional perspective view of a connecting portion;
FIG. 6 is a partially enlarged perspective view of the operating unit;
FIG. 7 is a perspective view of an end working portion;
FIG. 8 is an exploded perspective view of the end working portion;
FIG. 9 is a front view of a controller;
FIG. 10 is a block diagram of the controller;
FIG. 11 is a block diagram of a plurality of controllers and a host computer connected thereto via an LAN.
FIG. 12 is a chart showing contents of a usage history table;
FIG. 13 is a chart showing a connection information table displayed on a monitor;
FIG. 14 is a flow chart of processing procedures of the controller for controlling the manipulator;
FIG. 15 is a partially sectional perspective view of a manipulator according to an embodiment of the invention;
FIG. 16 is a flow chart of processing procedures of the controller for controlling the manipulator according to the embodiment;
FIG. 17 is a perspective view of a robotic surgical device according to a second embodiment of the present invention;
FIG. 18 is a view of a robotic surgical device according to a first modification example of the second embodiment;
FIG. 19 is a view of a robotic surgical device according to a second modification example of the second embodiment; and
FIG. 20 is a schematic perspective view of a surgical robotic system having the working unit connected to a distal end of a robotic arm.

### DESCRIPTION OF THE PREFERRED EMBODIMENTS

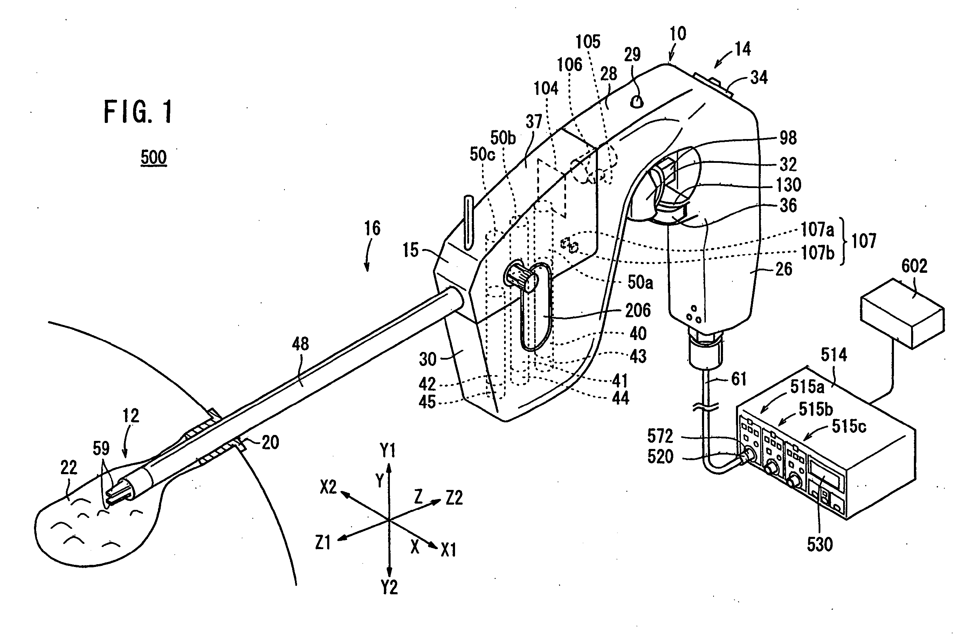

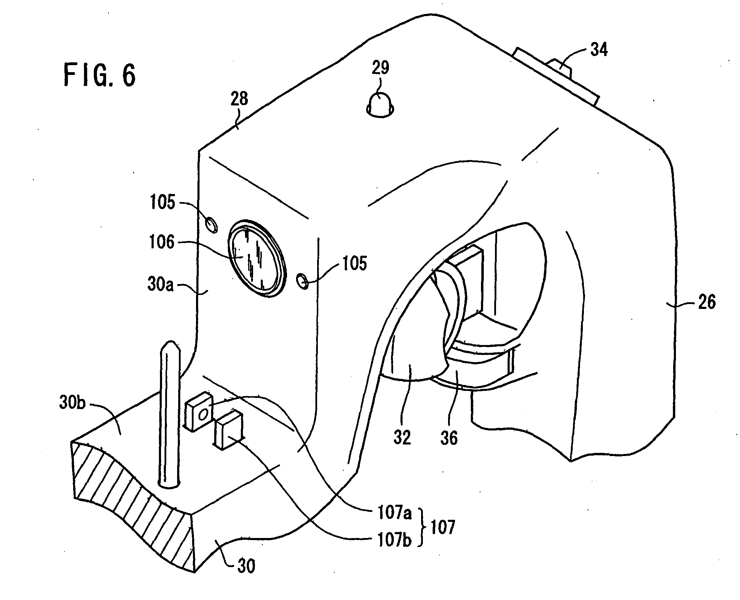

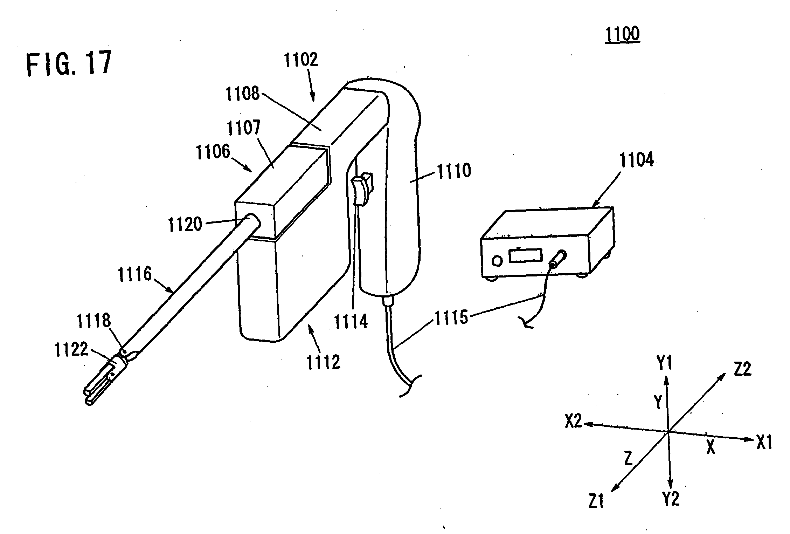

A medical manipulator system 500 according to an unclaimed example and embodiments of the present invention will be described below with reference to FIGS. 1 to 20. The manipulator system 500 shown in FIG. 1 is used in a laparoscopic operation, etc.

As shown in FIG. 1, the manipulator system 500 has a manipulator 10 and a controller 514.

A connector 520 is disposed in a connecting portion between the manipulator 10 and the controller 514 such that the manipulator 10 is removable from the controller 514.

The manipulator 10 is intended to grasp a part of a living body, a curved needle, or the like using an end working portion 12, thereby carrying out a predetermined procedure. The manipulator 10 has a basic structure containing an operating unit 14 and a working unit 16. The controller 514 electrically controls the manipulator 10, and is connected via the connector 520 to a cable 61 extending from the lower end of a grip handle 26.

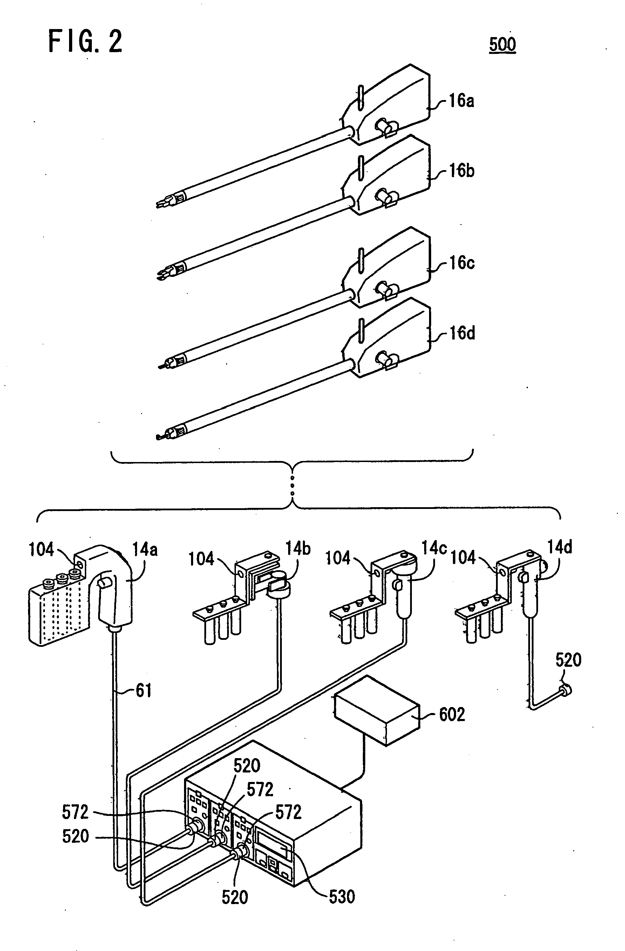

The controller 514 can simultaneously control three manipulators 10 independently. Sections of the controller 514 for controlling the first, second, and third manipulators 10 are referred to as a first port 515a, a second port 515b, and a third port 515c respectively. A host computer 602 shown in FIGS. 1 and 2 will be hereinafter described.

As shown in FIG. 2, the manipulator system 500 may have a variety of structures to be selected. Specifically, operating units 14a to 14d can be selectively used as the operating unit 14, and working units 16a to 16d can be selectively used as the working unit 16.

The operating unit 14b, 14c, or 14d instead of the operating unit 14a may be attached to the controller 514. Further, the working unit 16b, 16c, or 16d instead of the working unit 16a may be attached to each of the operating units 14a to 14d. Thus, an operator can select from combinations of the operating units 14a to 14d and the working units 16a to 16d depending on the type of the procedure, the procedure proficiency, etc. Among them, the working unit 16b has scissors as the end working portion 12. The working unit 16c has a blade-type electric surgical knife, and the working unit 16d has a hook-type electric surgical knife, as the end working portion 12. The working units 16a to 16d have the same arrangement of pulleys 50a, 50b, 50c (see FIG. 1) in a connecting portion 15.

The controller 514 can simultaneously control the three manipulators 10 as described above, and thus, three of the operating units 14a to 14d may be connected to the first port 515a, the second port 515b, and the third port 515c, respectively.

The manipulator 10 having the operating unit 14 and the working unit 16 will be described below.

The manipulator 10 is intended to grasp a part of a living body, a curved needle, or the like using an end working portion 12, thereby carrying out a predetermined procedure. The manipulator 10 is referred to typically as grasping forceps, a needle driver, etc.

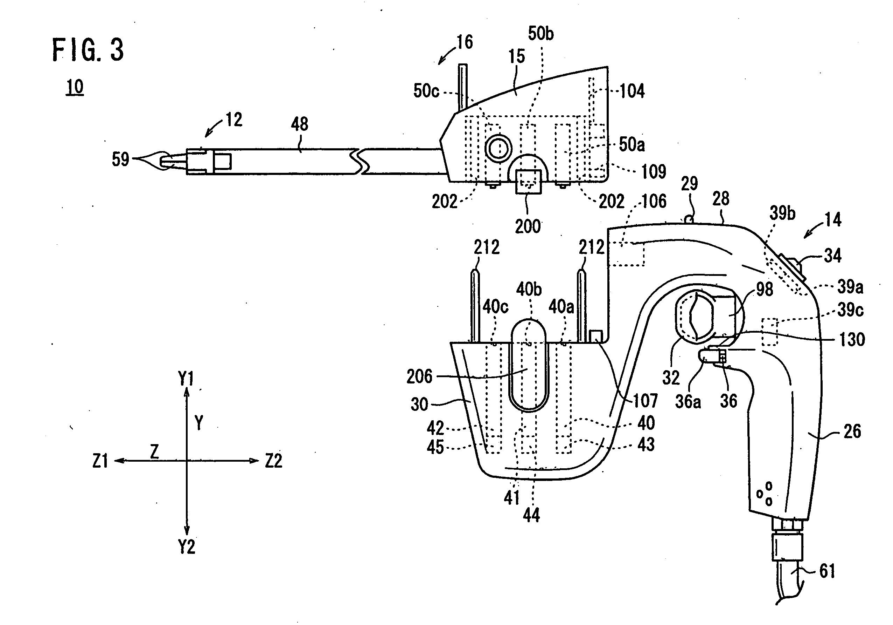

As shown in FIGS. 1 and 3, the manipulator 10 has the operating unit 14 grasped and handled by a hand, and the working unit 16 attachable to and detachable from the operating unit 14.

In the following description, in FIG. 1, the transverse direction of the manipulator 10 is referred to as X direction, the vertical direction is referred to as Y direction, and the extending direction of a connecting shaft 48 is referred to as Z direction. Further, the rightward direction is referred to as X1 direction, the leftward direction is referred to as X2 direction, the upward direction is referred to as Y1 direction, the downward direction is referred to as Y2 direction, the forward direction is referred to as Z1 direction, and the backward direction is referred to as Z2 direction. These expressions represent directions of the manipulator 10 when it is a neutral posture, unless otherwise noted. It should be noted that the expressions are used in the following description for illustrative purpose only, and the manipulator 10 may be used in any orientations, for example, the manipulator 10 may be used upside down.

The working unit 16 contains the end working portion 12 for carrying out a work, the connecting portion 15 which is connected to an actuator block (actuator unit) 30 of the operating unit 14, and the long, hollow connecting shaft 48 for connecting the end working portion 12 and the connecting portion 15. The working unit 16 can be separated from the operating unit 14 by performing a certain operation in the actuator block 30, and can be subjected to washing, sterilization, maintenance, etc. The actuator block 30, to which the working unit 16 is attached, is not limited to a portion containing therein motors 40, 41, 42, and the actuator block 30 also includes a contact surface 30a to a bridge 28.

The end working portion 12 and the connecting shaft 48 are thin, and thereby can be inserted through a cylindrical trocar 20 formed on an abdominal part of a patient, etc. into a body cavity 22. By controlling the operating unit 14, various procedures such as diseased part resection, grasp, suture, and tie-knot can be carried out in the body cavity 22.

The operating unit 14 contains the grip handle 26 that is grasped by a human hand, the bridge 28 extending from the upper part of the grip handle 26, and the actuator block 30 connected to one end of the bridge 28.

As shown in FIG. 1, the grip handle 26 of the operating unit 14 extends from the other end of the bridge 28 in the Y2 direction. The grip handle 26 has a length suitable for being grasped by a human hand, and has a trigger lever 32, a composite input part 34, and a switch 36, as an input means.

An LED (indicator) 29 is disposed in a visible position on the upper or side surface of the bridge 28. The LED 29 is an indicator for indicating the control state of the manipulator 10, and has such a size that the operator can easily recognize the indication and such a light weight that the handling of the manipulator 10 is not affected. In this unclaimed example, the LED 29 is disposed in an easily-visible position substantially at the center of the upper surface of the bridge 28.

The cable 61 connected to the controller 514 is disposed on the lower end of the grip handle 26. The grip handle 26 and the cable 61 may be connected integrally. The grip handle 26 and the cable 61 may be connected by a connector.

The composite input part 34 is a composite input means for transmitting a command of rotating the end working portion 12 in the roll direction (the axial rotation direction) or the yaw direction (the horizontal direction). For example, in the composite input part 34, a first input means may be axially rotated to transmit a command of rotating in the roll direction, and a second input means may be moved in the horizontal direction to transmit a command of rotating in the yaw direction. The trigger lever 32 is an input means for transmitting a command of opening and closing a gripper 59 (see FIG. 1) in the end working portion 12. The switch 36 is an input means for start-and-stop controlling the manipulator 10.

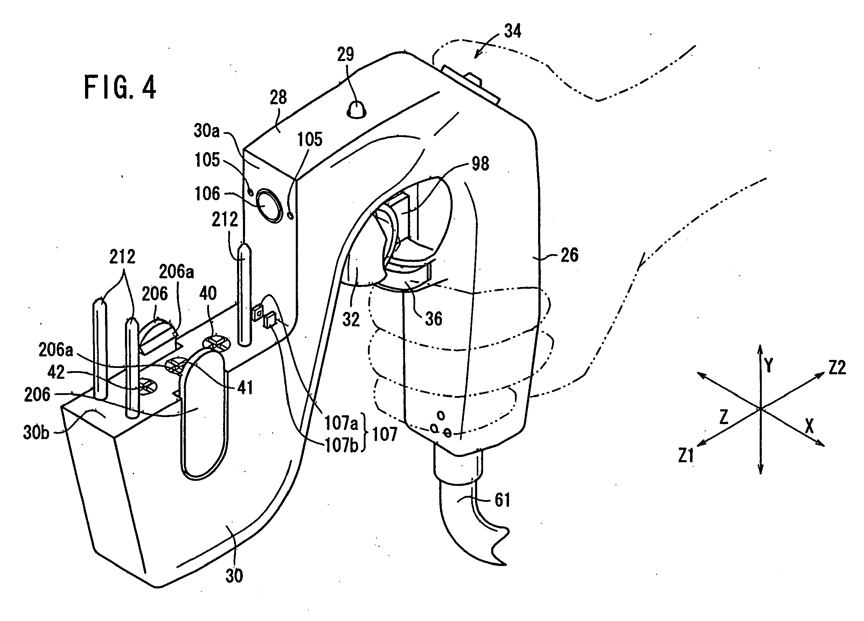

As shown in FIGS. 3 and 4, input sensors 39a, 39b, 39c are used for detecting the movement amounts of the composite input part 34 and the trigger lever 32 respectively. A movement signal (such as an analog signal) detected by the input sensors 39a, 39b, 39c is transmitted to the controller 514.

The trigger lever 32 projects in the Z1 direction on the lower side of the bridge 28. The position of the trigger lever 32 is such that it can be easily handled by an index finger.

The trigger lever 32 is connected via an arm 98 to the grip handle 26, and can be moved close to and away from the grip handle 26.

The switch 36 is an operation mechanism movable close to and away from the grip handle 26, and the trigger lever 32 and the switch 36 are arranged on the Z1 side of the grip handle 26 in the longitudinal direction of the grip handle 26 (the Y direction). The switch 36 is disposed immediately below the trigger lever 32 (on the Y2 side), and a thin plate 130 is placed between the switch 36 and the trigger lever 32.

The switch 36 is alternate, and by pushing an operation button 36a in the Z2 direction, the operation button 36a is fixed at a pushed position in the Z2 direction and the switch 36 is locked in an ON state. By pushing the switch 36 again, the switch 36 is changed from an ON state to an OFF state, and is moved toward the distal end in the Z1 direction by an elastic body (not shown), thereby returning to the initial position. The switch 36 can be kept in an ON or OFF state by these procedures, and it is not necessary to continue pushing the switch 36. Thus, the switch 36 only needs to be handled when the ON or OFF state is switched, and the trigger lever 32 can be handled at any time except when the ON/OFF state is switched. The switch 36 and the trigger lever 32 can be preferably used in combination in this manner.

The protrusion of the operation button 36a is different between the ON and OFF states, whereby the state of the switch 36 can be recognized by visually observing or touching the operation button 36a.

A mode of the manipulator 10 is changed by the switch 36. The mode is indicated by lighting of the LED 29 and a certain lamp in the controller 514. Specifically, the LED 29 and the lamp are in a green lighting state in a drive mode, and in a non-lighting state in a stop mode. Further, the LED 29 and the lamp are in a green blinking state during an automatically returning step and a reset step, and in the red blinking state during alarm generation.

The mode and operation of the manipulator 10 are changed by handling the switch 36. The state of the switch 36 is read by the controller 514. When the switch 36 is in an ON state, the manipulator 10 is changed to the drive mode by the controller 514. When the switch 36 is changed from the ON state to the OFF state, the motors 40, 41, 42 are automatically returned to the original points. Further, the manipulator 10 is changed to the stop mode after the motors 40, 41, 42 have been returned to the original points.

The drive mode is such a mode that the operating command of the operating unit 14 is effective for driving the motors 40, 41, 42. The stop mode is such a mode that the motors 40, 41, 42 are stopped regardless of the operating command of the operating unit 14. Further, the reset step is such a step that the motors 40, 41, 42 are automatically returned to the original points when a predetermined operation is carried out. The motors 40, 41, 42 are driven in the automatically returning step and the reset step regardless of the operating command of the operating unit 14, and thus in the steps, the manipulator 10 is in the automatic mode.

These modes and steps are separately controlled, and the lighting state of the LED 29 and lamp is changed, by the controller 514.

In the actuator block 30, the motors 40, 41, 42 corresponding to the three-degree-of-freedom mechanism of the end working portion 12 are arranged in the longitudinal direction of the connecting shaft 48. The motors 40, 41, 42 are small and thin, and the actuator block 30 has a compact flat shape. The actuator block 30 is disposed at the lower portion on the Z1 direction end of the operating unit 14. The motors 40, 41, 42 are rotated by the operating unit 14 under the control of the controller 514.

Angle sensors 43, 44, 45 are attached to the motors 40, 41, 42 to detect the rotation angles thereof. The detected angle signals are transmitted to the controller 514. For example, a rotary encoder is used as the angle sensors 43, 44, 45.

The working unit 16 contains the connecting portion 15 connected to the actuator block 30 and the hollow connecting shaft 48 extending from the connecting portion 15 in the Z1 direction. The pulleys 50a, 50b, 50c are rotatably disposed in the connecting portion 15, and are connected to drive shafts of the motors 40, 41, 42. The pulleys 50a to 50c each have a coupling.

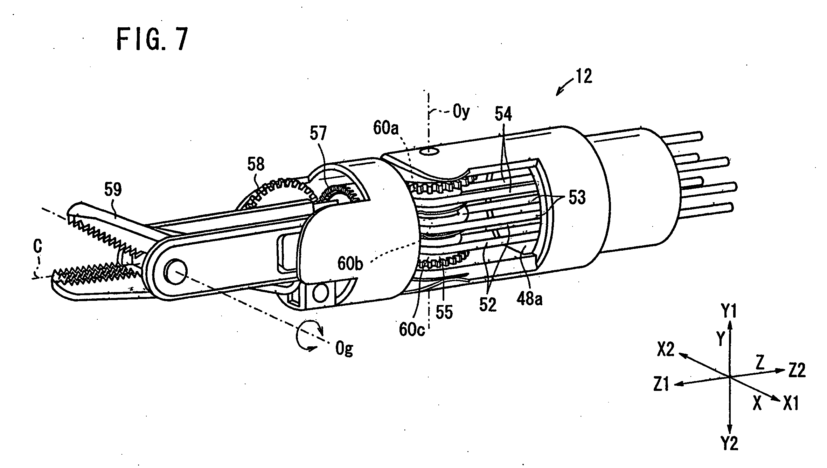

Wires 52, 53, 54 are wound around the pulleys 50a, 50b, 50c, and extend through a hollow part 48a (see FIG. 7) of the connecting shaft 48 to the end working portion 12. The types and diameters of the wires 52, 53, 54 may be the same.

The connecting portion 15 of the working unit 16 can be separated from the operating unit 14 by performing a predetermined operation in the actuator block 30, and can be subjected to washing, sterilization, maintenance, etc. The working unit 16 can be changed with another working unit, and the length of the connecting shaft 48 and the mechanism of the end working portion 12 may be selected depending on an intended procedure.

The structure of the connecting portion 15 is such that rotary shafts 40a, 40b, 40c of the motors 40, 41, 42 are fitted into center holes of the pulleys 50a, 50b, 50c. The pulleys 50a, 50b, 50c each have a cross-shaped connecting projection at the lower end in the Y2 direction, and the rotary shafts 40a, 40b, 40c each have a cross-shaped connecting recess. The connecting projections and the connecting recesses are engageable with each other, and thereby the rotary motions of the motors 40, 41, 42 are reliably transmitted to the pulleys 50a, 50b, 50c. The connecting projections and the connecting recesses are not engaged with each other when the pulleys 50a, 50b, 50c and the motors 40, 41, 42 are not in the original points. The shapes of these engaging portions are not limited to the cross shape.

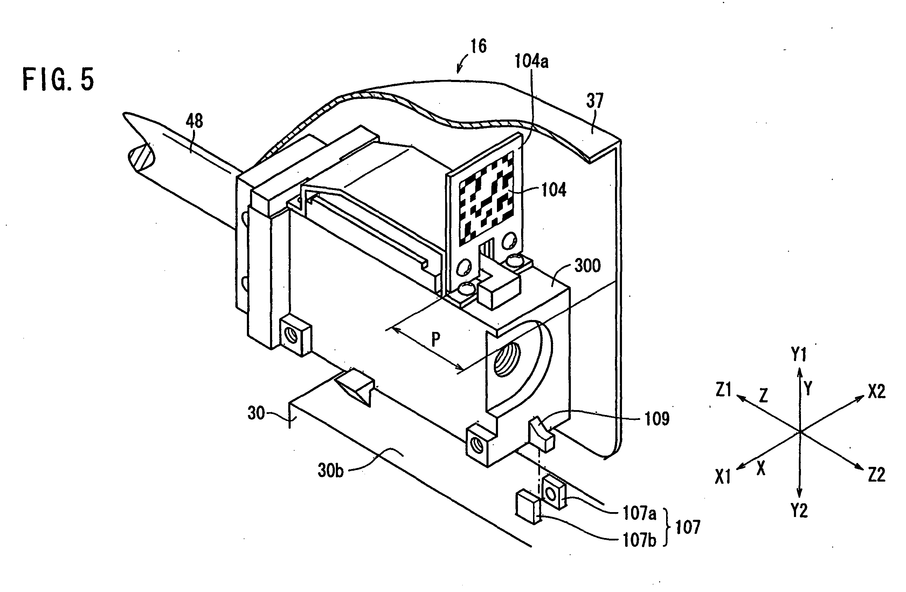

A camera (an ID recognizer or an image-capturing means) 106 and two white LEDs (a lighting means) 105 are disposed on the contact surface 30a between the actuator block 30 and the bridge 28. The contact surface 30a is an X-Y plane, which is brought into contact with an end surface of a cover 37 in the connecting portion 15 of the working unit 16. The camera 106 is for capturing an image of an image code 104 as described later (see FIG. 5), and may be a CCD or CMOS camera. The white LEDs 105 have an optical axis in the direction of illuminating the image code 104, so that the image code 104 can be recognized more reliably by the camera 106. The white LEDs 105 are horizontally symmetrically disposed with reference to the camera 106, and thereby the image code 104 can be irradiated with a balanced light. The white LEDs 105 may be vertically arranged with the camera 106 interposed therebetween, and three or more LEDs 105 may be disposed at equal intervals. Only one LED 105 may be used as long as it has a sufficient light intensity.

On an upper surface 30b of the actuator block 30, on which the connecting portion 15 is mounted, a working unit detecting means 107 for detecting the presence of the connecting portion 15 is disposed in the vicinity of the Z2 direction end. The working unit detecting means 107 has a phototransmitter 107a and a photoreceiver 107b facing each other. When a detection piece 109 disposed at the rear end of the connecting portion 15 is inserted between the phototransmitter 107a and photoreceiver 107b and blocks a light from the phototransmitter 107a, the attachment of the connecting portion 15 is detected by the working unit detecting means 107. The phototransmitter 107a and the photoreceiver 107b are arranged facing each other in the X direction and being close to each other. For example, the phototransmitter 107a may be an LED, and the photoreceiver 107b may be a photodiode.

As shown in FIG. 5, on the upper surface of a pulley container 300 in the connecting portion 15, a two-dimensional image code (an ID holder) 104 is placed in the vicinity of the Z2 direction end. For example, the image code 104 has a substantially square matrix shape, and black and white patterns are printed on the divisions thereof. The image code 104 is attached to a plate 104a in the X-Y plane, and is positioned at an appropriate distance P from the rear end of the cover 37 in the forward Z1 direction.

The image code 104 contains information of the working unit 16, such as the identification data, specification, time stamp (manufacturing date, etc.), serial number, and usage count upper limit. The identification data of the working unit 16, which is included in the image code 104, has a different value, so as to identify the working units.

The connecting portion 15 may have a plurality of image codes 104. In the case of using two image codes 104, one may have individual specific information such as the identification data, manufacturing date, and serial number, and the other may have common model information such as the specification or usage count upper limit.

The image code 104 is not limited to the two-dimensional data, and may be one-dimensional bar code. The colors of the patterns printed on the divisions of the image code 104 are not limited to black and white, and may be an infrared absorbing color and an infrared reflecting color, or three or more colors for indicating information based on difference in color.

The information in the image code 104 is read by the controller 514, and shown in a working state display 530 (see FIG. 1). Alternatively the read information may be judged, and a caution or warning may be shown on the working state display 530.

The small detection piece 109 projects backward in the lower end on the rear surface of the pulley container 300. When the connecting portion 15 is attached to the actuator block 30, the detection piece 109 is inserted between the phototransmitter 107a and photoreceiver 107b, and blocks a light form the phototransmitter 107a, and the image code 104 faces the camera 106 at the focal distance P.

At the time, the image code 104 and the camera 106 are covered in a substantially closed region with the cover 37. Thus, contamination of the image code 104 and the camera 106 can be prevented, and the image can be stably captured while blocking ambient light. Even when the image code 104 and the camera 106 are in the closed region, the image can be stably taken under the lighting by the white LEDs 105. The cover 37 for covering the image code 104 and the camera 106 may be disposed on the actuator block 30. The relative positions and orientations of the image code 104 and the camera 106 are fixed, so that the camera 106 need not recognize the position and orientation of the image code 104. Therefore, a code for recognizing the position and orientation is not required or is reduced, whereby the amount of information that can be included in the image code 104 can be increased.

Whether the working unit 16 is attached to the actuator block 30 or not can be detected by the controller 514 using the working unit detecting means 107. When the working unit 16 is attached to the actuator block 30 (e.g. at the time point of the attachment, or in the period between the attachment and the substantial start of the procedure), the controller 514 controls the camera 106 and the white LEDs 105 to receive the identification signal from the image code 104. Thus, the controller 514 only needs to receive the identification signal when the working unit 16 is attached to the actuator block 30, and the camera 106 and the white LEDs 105 can be stopped at any time except when the working unit 16 is attached to the actuator block 30, whereby the processing load and the power consumption can be reduced.

A visible light, an infrared light, etc. can be used for capturing an image by the camera 106. In the case of using the infrared light, the image of the image code 104 can be clearly captured even in a dark place. In this case, the image code 104 may be irradiated with an infrared ray LED.

The working unit detecting means 107 is not limited to the above structure having the phototransmitter 107a and the photoreceiver 107b, and for example may be a limit switch, which is operated by the detection piece 109. The controller 514 can receive the identification data of the working unit 16 and control the working unit 16 based on the identification data in accordance with the type of the working unit 16.

It is not necessary to directly applying electricity to the image code 104, and the connecting portion 15 and the working unit 16 have no electrical contacts and no electricity storages such as batteries. Thus, the working unit 16 detached from the operating unit 14 can be easily washed and sterilized. All electric devices such as motors, switches, and sensors are placed in the operating unit 14, while the mechanical components such as the connecting shaft 48 and the end working portion 12 are placed in the working unit 16, so that the washing efficiency of the working unit 16 is improved. It is preferred that the working unit 16 and the operating unit 14 are separately maintained and washed because the contamination degrees, contamination types, and washing methods are different therebetween.

In order to detach the connecting portion 15 from the operating unit 14, levers 206 formed on both sides of the actuator block 30 are pressed to be tilted outward and then wedges 206a of the levers 206 are disengaged from engaging pieces 200 formed on both sides of the connecting portion 15. Then, the connecting portion 15 is pulled out upward (in the Y1 direction) and detached from the operating unit 14. Three alignment pins 212 are disposed on the upper surface of the actuator block 30, and are fitted into mating holes 202 formed on the connecting portion 15 to stably fix the connecting portion 15. When the connecting portion 15 is attached to the operating unit 14, the connecting portion 15 is moved downward (in the Y2 direction) while the three alignment pins 212 is kept at positions for fitting into the mating holes 202. Thus, the levers 206 are moved outward and then return to the original positions, and thereby are engaged with the engaging pieces 200 to complete the attachment.

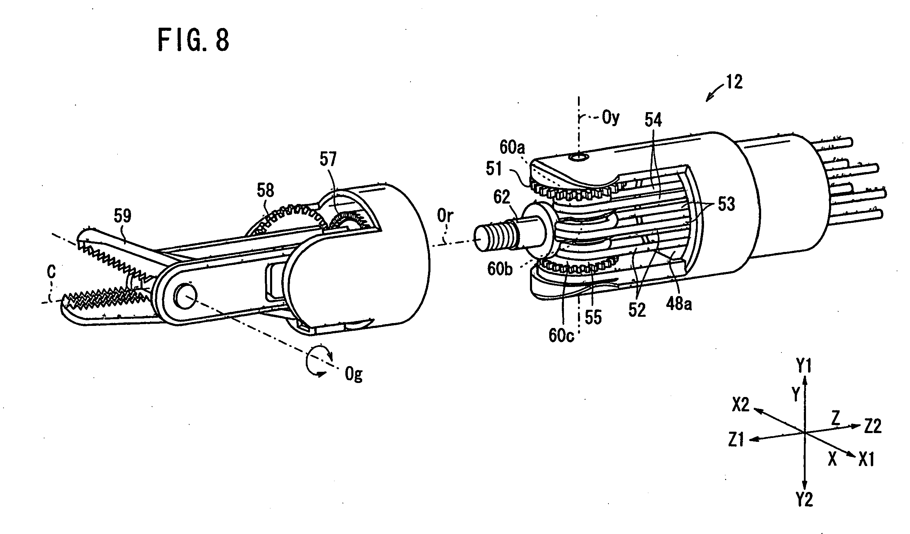

As shown in FIGS. 7 and 8, the end working portion 12 has a three-degree-of-freedom mechanism, which contains a mechanism (a tilt mechanism or a pivot shaft) having a first degree of freedom for rotating a portion of the end working portion 12 that is positioned ahead of a first rotary axis Oy extending in the Y direction, in the yaw directions about the first rotary axis Oy, a mechanism (a roll rotation mechanism) having a second degree of freedom for rotating the portion of the end working portion 12 in the roll direction about a second rotary axis Or, and a mechanism having a third degree of freedom for opening and closing the gripper 59 at the distal end about a third rotary axis Og.

The first rotary axis Oy of the mechanism having a first degree of freedom may be rotatable and non-parallel to an axis C extending from the proximal end to the distal end of the connecting shaft 48. The second rotary axis Or of the mechanism having a second degree of freedom may be rotatable and parallel to the extending axis of the distal end of the end working portion 12 (the gripper 59), the distal end being rotatable.

The end working portion 12 is driven by the wires 52, 53, 54, which are each wound around a corresponding cylinder 60c, 60b, 60a.

In the end working portion 12, gears 51, 55 are rotated by the wires 52, 54, whereby a face gear (not shown) is rotated, and then the distal end can be rotated in the roll direction. The gear 51 is rotated by the wire 54, whereby the gripper 59 can be opened and closed via the face gear 57 and the gear 58. Further, the distal end can be rotated in the yaw direction by the wires 52, 53, 54 via a main shaft member 62.

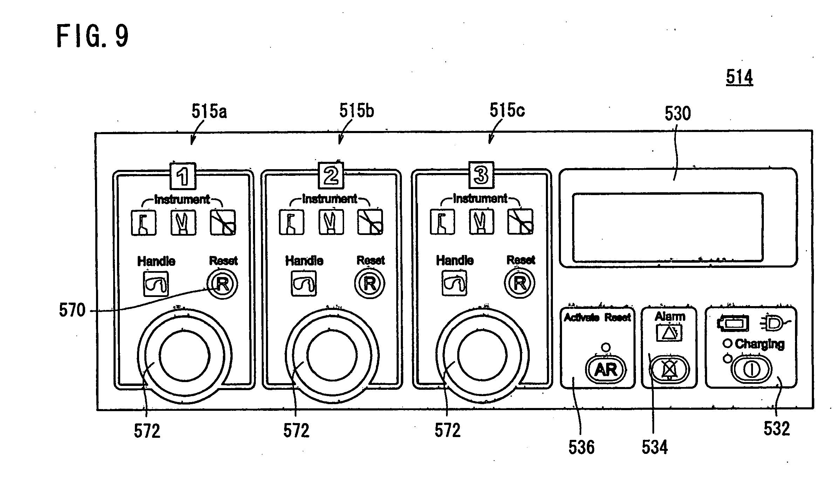

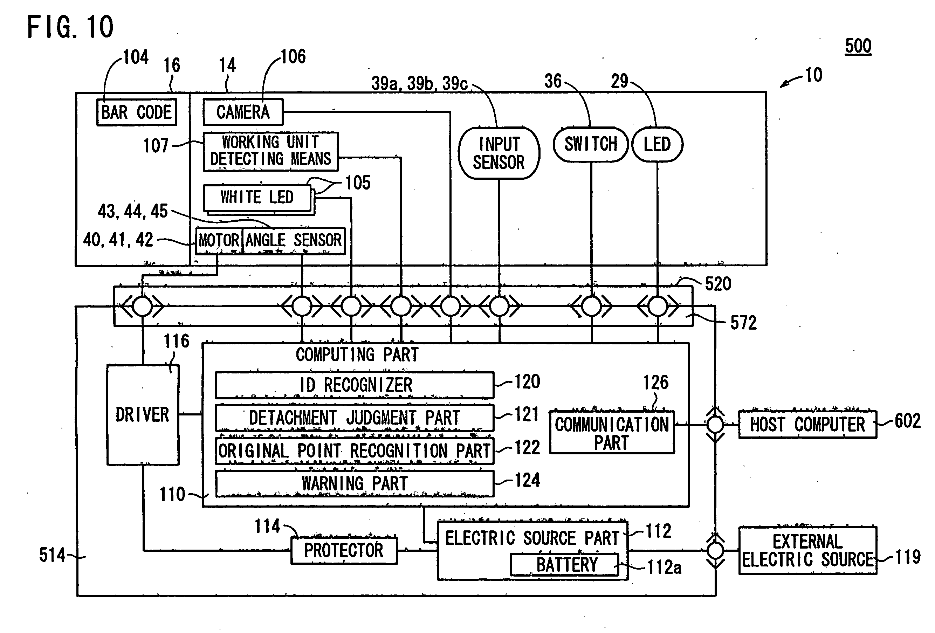

The controller 514 will be described below with reference to FIGS. 9 and 10.

As shown in FIG. 9, the working state display 530, an electric source information display 532, an alarm part 534, an activate reset part 536, the first port 515a, the second port 515b, and the third port 515c are disposed on the front face of the controller 514.

The working state display 530 has a liquid crystal display screen for showing the working state or command of the manipulator 10 in order that an operator, an operation assistant, or the like can easily recognize the operating state.

The first port 515a, the second port 515b, and the third port 515c each have a receptacle connector 572, to which the manipulator 10 can be connected, and each have an information display and a reset switch 570.

The internal structure of the controller 514 will be described below with reference to FIG. 10. For purposes of simplifying the description, in FIG. 10, only the components for the first port 515a are shown, and part of the components for the second port 515b and the third port 515c are omitted. Among the components for the second port 515b and the third port 515c, some components such as a computing part 110 are common to the ports 515a, 515b, 515c and the other components such as a driver 116 are independent. The surface structure of the controller 514 (see FIG. 9) is omitted in FIG. 10.

As shown in FIG. 10, the controller 514 has the computing part 110, an electric source part 112, a protector 114, and the driver 116. In the electric source part 112, an electric power from an external electric source 119 is controlled and applied to each component, and a battery 112a is charged. When the electric power is not supplied from the external electric source 119, the electric power supply of the external electric source 119 is automatically changed to the electric power supply of the battery 112a by the electric source part 112. Thus, the electric source part 112 acts as a so-called uninterruptible electric source. Normally, the battery 112a is connected in parallel to an internal transformer rectifier unit.

The electric power application to the manipulator 10 is stopped by the protector 114 based on information such as operation period information, driver information, and a stop command in the computing part 110. The manipulator 10 can be immediately stopped by blocking the electric power for the driver 116 using the protector 114.

The computing part 110 is connected to the angle sensors 43, 44, 45, the input sensors 39a, 39b, 39c, and the switch 36. In the computing part 110, based on signals from these components, the operation of the manipulator 10 is determined, a command signal is transmitted to the driver 116, and the state of the manipulator 10 is shown on the working state display 530. The computing part 110 is further connected to the LED 29, and controls the lighting of the LED 29.

The computing part 110 is connected to the camera 106 and the white LEDs 105, and controls the capturing and lighting.

Further, the computing part 110 is connected to and controls each switch and lamp of the working state display 530, the electric source information display 532, the alarm part 534, the activate reset part 536, the first port 515a, the second port 515b, and the third port 515c disposed on the controller 514 (see FIG. 9). The computing part 110 contains a CPU, an ROM, an RAM, etc., and reads and executes a program to perform a software process.

The driver 116 is connected to the motors 40, 41, 42, and the motors 40, 41, 42 are driven based on a command from the computing part 110. In a driving system of the motors 40, 41, 42, a movement angle command value for the end working portion is determined from the input sensors 39a, 39b, 39c, an angle signal is obtained from the angle sensors 43, 44, 45, the deviation between the movement angle command value and the angle signal is determined, a compensation process is carried out based the deviation, and a command signal is transmitted to the driver 116. Thus, the driving system of the motors 40, 41, 42 forms a closed loop.

The computing part 110 has an ID recognizer 120, a detachment judgment part 121, an original point recognition part 122, a warning part 124, and a communication part 126. The ID recognizer 120 can recognize the identification data in the image code 104 via the camera 106. In the detachment judgment part 121, the identity of the working unit 16 is judged based on the identification data recognized by the ID recognizer 120 when the working unit 16 is attached to or detached from the actuator block 30 of the operating unit 14.

In the trigger lever 32 and the composite input part 34 (see FIG. 1), a predetermined voltage is applied to each of the input sensors 39a, 39b, 39c (such as potentiometers) for detecting the operation amount by a human hand, and a range of the voltage is set as an operation range. Thus, each of the trigger lever 32 and the composite input part 34 can be used not only as an operation input means but also a recognition means for detecting the detachment of the operating unit 14.

The original point recognition part 122 recognizes whether the end working portion 12 is in the predetermined original point or not, based on a signal from the angle sensors 43, 44, 45. When the end working portion 12 is judged to be not in the original point by the original point recognition part 122, if the working unit 16 is judged to be detached from the operating unit 14 based on a signal from the working unit detecting means 107, a detachment warning is provided by the warning part 124.

Further, while providing the detachment warning, the warning part 124 monitors identification data obtained from the ID recognizer 120 and recognizes that a working unit 16 is reattached. In this case, if the obtained identification data is identical with identification data that was recognized before detachment, the warning part 124 stops the detachment warning. If the obtained identification data is different from identification data that was recognized before detachment, an improper connection warning is provided by the warning part 124.

The detachment warning and the improper connection warning may be provided as a sound or voice, and also may be shown as a message in the working state display 530. It is preferred that the detachment warning and the improper connection warning can be easily distinguished. In the case of using the sound or voice, for example, the warnings may be provided as buzzer sounds with different blowing intervals or frequencies.

The communication part 126 is connected to an external LAN (Local Area Network) 600, and information can be sent and received therebetween.

When the working unit 16 is judged to be detached by the working unit detecting means 107, or the end working portion 12 is judged to be in the original point by the original point recognition part 122, the electricity supply to the driver 116 is stopped by the computing part 110 via the protector 114.

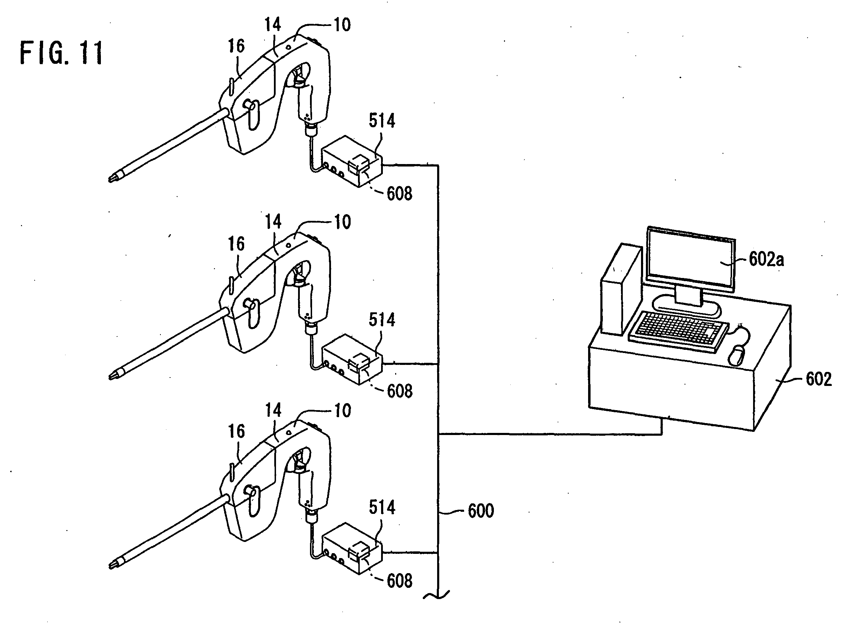

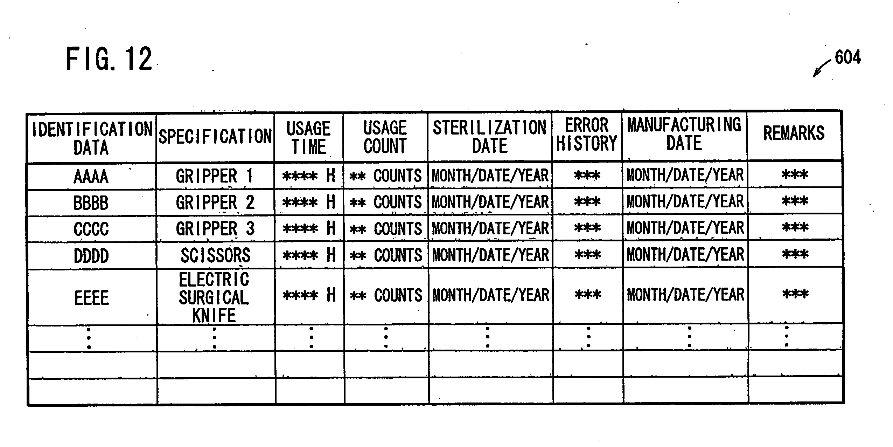

As shown in FIG. 11, a plurality of (e.g. three) controllers 514 may be connected to the LAN 600. A usage history management means of the host computer 602 is connected to the LAN 600. In the host computer 602, a usage history table 604 shown in FIG. 12 is recorded on an internal recording means. A usage history data corresponding to an identification number is sent and received between the host computer 602 and each of the controllers 514, and is managed by the host computer 602. The host computer 602 may have such a structure as separated from the controllers 514, and one of the controllers 514 may have the usage history table 604 and act as the host computer 602.

The LAN 600 may be a wired or wireless means, a means using an electric source line, etc., and may be replaced with another communication means. The LAN 600 is not limited to a local network, and may be, for example, a network shared by a plurality of medical facilities. The communication means may be such that the host computer 602 is prepared by a manipulator manufacturer, each controller 514 is connected to the host computer 602 over a common network, and the usage history is managed by the manufacturer.

As shown in FIG. 12, the usage history table 604 has columns for the identification data, specification, usage time, usage count, sterilization date, error history, manufacturing date, and remarks. Further, individual information such as a corrected phase value (or an corrected original point value) may be recorded on the usage history table 604. The identification data of the working unit 16 is shown in the identification data column. The specification of the working unit 16, particularly the type of the end working portion 12, the length of the connecting shaft 48, etc., is shown in the specification column. The number of using a corresponding working unit 16 is shown in the usage count column. The date of last sterilizing the working unit 16 is shown in the sterilization date column. The history information of an error generated in a corresponding working unit 16 is shown in the error history column. The manufacturing date of a corresponding working unit 16 is shown in the manufacturing date column. Other optional information can be written on the remarks column.

The information in the sterilization date column and the remarks column are input by an input means such as a keyboard or a mouse in the host computer 602.

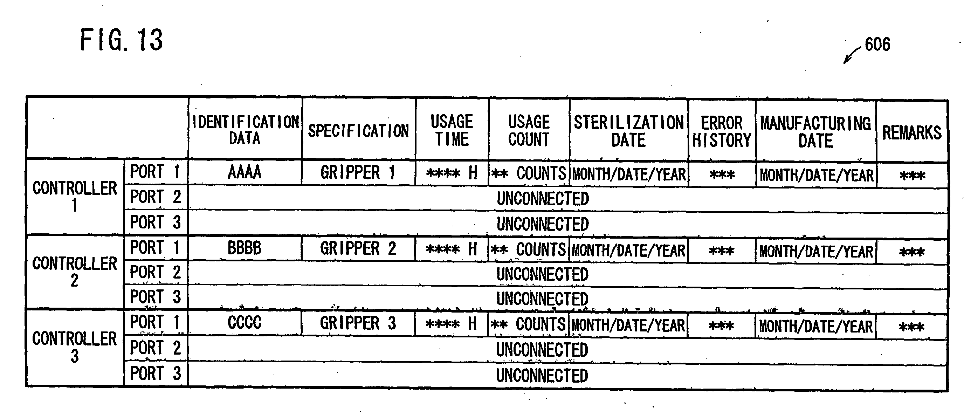

As shown in FIG. 13, a connection information table 606 is shown on a monitor 602a of the host computer 602. Information on connection ports 1 to 3 of the three controllers 514, which are connected to the host computer 602, are shown in the connection information table 606. The usage history data of the connected ports are shown in a corresponding section of the connection information table 606 with reference to the usage history table 604. The unconnected ports are shown as "unconnected".

When the working unit 16 is attached to the actuator block 30, based on a signal from the working unit detecting means 107, each controller 514 controls the camera 106 and the white LEDs 105, receives the identification signal of the image code 104, and receives the usage history data corresponding to the identification signal from the host computer 602. Further, after the working unit 16 is detached from the actuator block 30, the usage history data is updated by each controller 514, and is transmitted to the host computer 602. Then, the host computer 602 records the obtained usage history data on the usage history table 604, based on the identification signal.

The position of the usage history management means is not limited to the host computer 602, and a management part 608 having the same function as the usage history management means may be disposed in one or all of the controllers 514 as shown by a dotted line in FIG. 11.

When the management part 608 is disposed in one of the controllers 514, information are sent and received between the management part 608 and the other controllers 514 to centrally manage the usage history table 604 in the same manner as the host computer 602.

When the management part 608 is disposed in all the controllers 514, one management part 608 is made effective by operating a selecting switch, the other management parts 608 being ineffective.

Further, when the management part 608 is disposed in all the controllers 514, the information in the usage history table 604 may be dispersion-managed. For example, when a certain working unit 16 is connected to one of the controllers 514, the controller 514 may communicate with all the other controllers 514 and obtain, from all the other controllers 514, the current usage history data corresponding to the identification data of the connected working unit 16. Then, the information of the working unit 16 may be updated and managed in the management part 608 of the connected controller 514. In controllers 514 that have transmitted the information to the controller 514, from which the current usage history data is transmitted, the information may be deleted or flagged as a sign of transmitted data.

The usage history data of the working unit 16 may be updated and managed in the controller 514 to which the working unit 16 is connected most recently (or the working unit 16 is being connected). Further, the usage history data may be updated and managed in the controller 514 to which the working unit 16 is connected first, and may be redundantly managed in all the controllers 514.

When the information of the usage history table 604 and the connection information table 606 is displayed, the monitor 602a may be connected to the controller 514. Alternatively, the host computer 602 may be connected as a display device to the LAN 600.

The information processing procedures for the identification data in the manipulator system 500 will be described below. The following procedures are carried out in the controller 514 unless otherwise noted.

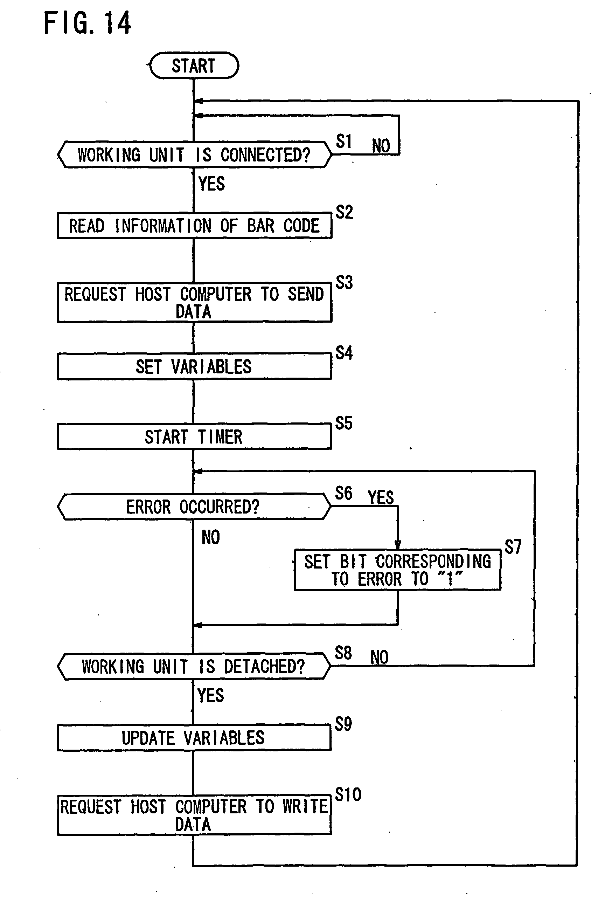

In the step S1 of FIG. 14, the controller 514 receives a signal from the working unit detecting means 107, to confirm whether the working unit 16 is connected or not to the actuator block 30 of the operating unit 14. When the working unit 16 is not connected, the controller 514 is kept in the standby state, and when the working unit 16 is connected, control goes to step S2.

In the step S2, the camera 106 and the white LEDs 105 are controlled to read the information of the image code 104. The information may include the identification data, specification, and manufacturing date. After the information is obtained, the camera 106 and the white LEDs 105 are stopped.

In the step S3, the identification data and information on the connected port are transmitted from the controller 514 to the host computer 602, and the controller 514 requests the host computer 602 to send data. The usage history table 604 is searched by the host computer 602 based on the transmitted identification data, and the corresponding usage history data is transmitted from the host computer 602 to the controller 514. In the host computer 602, the usage history data corresponding to the identification data is shown in the corresponding column of the connection information table 606.

In a case where the identification data is not contained in the usage history table 604 (i.e. a novel working unit 16 is connected), this information is transmitted to the controller 514 and another column for the identification data is formed in the usage history table 604.

In the step S4, based on the information from the host computer 602, the usage time data is assigned to a variable Hr, the usage count data is assigned to a variable No, and the error history data is assigned to a variable Er. In the case where the identification data is not stored in the host computer 602 and this information is transmitted to the controller 514, the value 0 is assigned to each of the variables Hr, No, and Er. The variable Er may be a binary number data having a bit number representing the number of error types.

In the step S5, a timer is started to measure the operating time ΔHr of the connected working unit 16. The operating time ΔHr may be measured only when the working unit 16 is in the drive mode.

In the step S6, an error detecting process is carried out. The step S7 is carried out when an error occurs, and the step S8 is carried out when errors do not occur.

In the step S7, a process appropriate for the generated error is carried out, and the bit corresponding to the error is set to "1" in the variable Er. After the setting, the history of the error occurrence can be recognized by referring to the bit. Then, the step S8 is carried out.

In the step S8, a signal is obtained from the working unit detecting means 107, to confirm whether the working unit 16 is detached from the actuator block 30 of the operating unit 14 or not. The next step S9 is carried out when the working unit 16 is detached, and the step S6 is carried out again when the working unit 16 is not detached.

In the step S9, when the timer is stopped, the operating time ΔHr is obtained, and the variable Hr is updated in the manner of Hr←Hr+ΔHr. Further, the variable No is incremented in the manner of No←No+1.

In the step S10, the identification data of the detached working unit 16, the information on the detached port, and the variables No, Hr, and Er are transmitted from the controller 514 to the host computer 602, and the controller 514 requests the host computer 602 to write data. The usage history table 604 is searched by the host computer 602 based on the transmitted identification data, to write the corresponding usage history data. Therefore, even when the host computer 602 is requested to send data by the three controllers 514, the correct usage history data corresponding to the identification data of the working unit 16 can be transmitted from the host computer 602. Then, the system is returned to the step S1.

In the host computer 602, the information of the corresponding column in the connection information table 606 is hidden, and the sign "unconnected" is displayed.

As described above, in the manipulator system 500, the image code 104 and the camera 106 are not in contact with each other, so that the identification data of the working unit 16 can be transmitted without electric powers and electric contacts, and the working unit 16 can be easily washed and sterilized. Further, communication errors are not caused owing to the absence of electrical contacts. Electrically noncontact type devices are generally more durable than contact type devices.

Various information are shown as image information in the image code 104, and thereby contactless communication can be easily achieved.

The controllers 514 are connected to the host computer 602 via the LAN 600, and can access the usage history table 604. Therefore, the usage history can be preferably obtained regardless of which controller 514 is connected to the working unit 16.

The image code 104 is positioned in the vicinity of the rear end of the connecting portion 15, whereby the distance between the image code 104 and the camera 106 can be short, and the identification data is reliably transmitted.

The position of the end working portion 12 is calculated using e.g. the original position as a reference. Therefore, when the working unit 16 is changed with another one during an operation, it is desirable that the working unit 16 is detached after the end working portion 12 and the pulleys 50a to 50c of the working unit 16 are precisely positioned at axial positions corresponding to the original points. When the working unit 16 is detached while they are not placed at the positions corresponding to the original points, a certain alarm may be sounded for the working unit 16 to be reattached. In this case, the working unit 16 needs to be identified to prevent the attachment of another working unit. The working unit 16 can be identified based on the identification data from the image code 104, and when another working unit is attached, a certain alarm may be sounded.

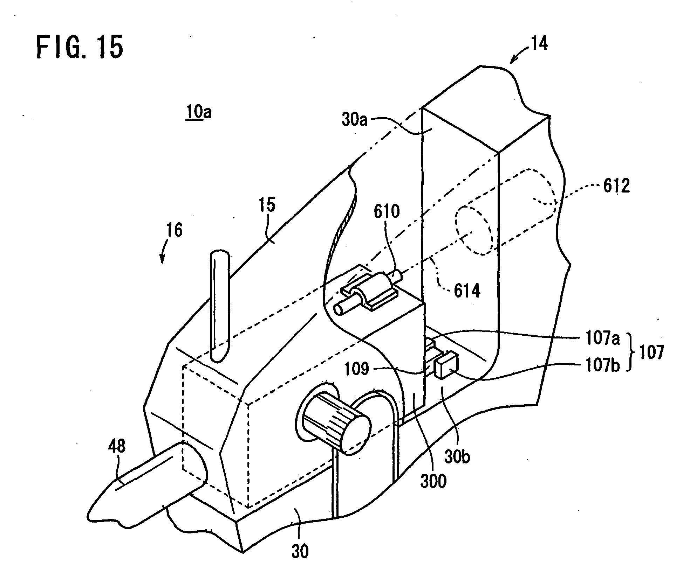

A manipulator 10a according to an embodiment of the invention will be described below with reference to FIG. 15. In the manipulator 10a, the same components as the manipulator 10 are represented by the same numerals, and explanations therefor are omitted. In the manipulator 10a, an RFID 610 and a transmitter-receiver 612 are used instead of the image code 104 and the camera 106, and the white LEDs 105 are removed.

As shown in FIG. 15, the RFID (Radio Frequency Identification, an ID holder) 610 is disposed in the vicinity of the rear end of the connecting portion 15 in the manipulator 10a. The RFID is a wireless authentication system, which has a small IC chip containing the product identification data and wirelessly reads or updates information. The RFID is referred to also as a wireless tag, IC tag, or µ chip. The RFID 610 contains information equal to those of the above described image code 104, i.e. the information such as the identification data, specification, time stamp (manufacturing date, etc.), serial number, and usage count upper limit of the working unit 16. The RFID 610 is recordable, and individual information such as the usage count, usage time, error history, sterilization date, and corrected phase value (or corrected original point value) are recorded thereon.

In the actuator block 30, the transmitter-receiver (an ID recognizer, data transmitter) 612 is disposed such that it faces the RFID 610 when the connecting portion 15 is connected to the actuator block 30. Information is sent and received between the transmitter-receiver 612 and the RFID 610 via a radio wave. The relative positions and orientations of the RFID 610 and the transmitter-receiver 612 are fixed when the connecting portion 15 is attached to the actuator block 30. Therefore, the radio wave directivity of each of the RFID 610 and the transmitter-receiver 612 is narrowed, and the main lobe of the directivity is directed toward the opposite component to strengthen the radio wave in the direction, and thus, the sending and receiving can be reliably carried out under reduced electric power. The radio wave is weakened in directions other than the main lobe direction, and thus the radio wave is not radiated uselessly over a wide range, resulting in no possibility of interference. In FIG. 15, a two-dotted line 614 represents an axis, on which the strength and sensitivity of the radio wave transmitted and received between the RFID 610 and the transmitter-receiver 612 are largest. It is preferred that a direction of the RFID 610 with the strongest directivity is completely aligned with a direction of the transmitter-receiver 612 with the strongest directivity. Practically, in the main lobe beam width, -3 dB power patterns of the RFID 610 and the transmitter-receiver 612 may be directed to each other, for example.

The RFID 610 and the transmitter-receiver 612 are remarkably close to each other. Such a short-distance system with small energy results in a reduced electric power consumption and no possibility of interference.

For example, a 13.56-MHz-band, 2.45-GHz-band, or 5-GHz-band RFID can be suitably used in such a system, in which the RFID 610 and the transmitter-receiver 612 are disposed in the fixed relative positions at a small distance.

The controller 514 controls the manipulator 10a in accordance with the RFID 610 and the transmitter-receiver 612, although the detailed description therefor is omitted. The RFID is recordable, and data such as the usage count, usage time, error history, and sterilization date may be written thereon. A rewriting preventing means may be used to prevent the data such as the identification data, serial number, and specification in the RFID from being carelessly changed.

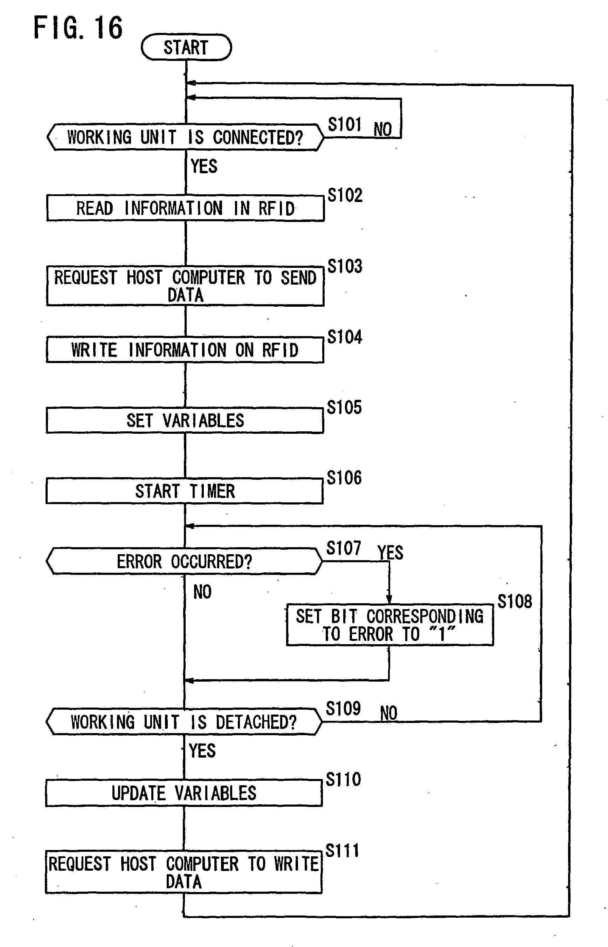

The controller 514 controls the manipulator 10a as shown in FIG. 16. The steps S101 to S103 of FIG. 16 correspond to the steps S1 to S3 of FIG. 14, and the steps S105 to S111 of FIG. 16 correspond to the steps S4 to S10 of FIG. 14. The step S102 is different from the step S2 in that the information is read from the RFID 610, instead of the image code 104.

The step S104 is carried out between the steps S103 and S105. Information is written on the RFID 610 in the step S104. The information contains all the corresponding usage history data in the usage history table 604 of the host computer 602 (see FIG. 12).

In the manipulator 10a according to the embodiment, the RFID 610 and the transmitter-receiver 612 are not in contact with each other, and the identification data of the working unit 16 can be obtained without electrical contacts and electricity storages such as batteries. Thus, the working unit 16 can be easily washed and sterilized. Signals can be transmitted between the RFID 610 and the transmitter-receiver 612 through a radio wave, and thereby contactless communication can be easily achieved. The RFID 610 has a small, simple structure capable of easily communicating. The usage history data is received to and recorded on the RFID 610, and the usage history of each working unit 16 can be easily managed.

The transmitter-receiver 612 may be an antenna. An electrical circuit, which is connected to the transmitter-receiver 612, may be disposed in the operating unit 14 or the controller 514.

The identification data may be contactless-transmitted between the working unit 16 and the operating unit 14 through a magnetism or light (e.g. infrared communication), other than the image information of the image code 104 and the radio wave of the RFID 610. The working unit 16 can be easily cleaned and washed also in this case.

Although the above described manipulator 10 is intended for medical use, the manipulator can be suitably used, for example, in repairing a narrow portion of an energy device, etc. or in a remote operation mechanism for operating a patient from a distance using a telecommunication means, etc.

Then, a medical manipulator system 1100 according to a second embodiment will be described below. First, components of the medical manipulator system 1100 and the corresponding components of the above mentioned manipulator system 500 will be described.

The main components of the medical manipulator system 1100: a manipulator 1102 (a), a control unit 1104 (b), a surgical instrument 1106 (c), a surgical instrument control unit 1112 (d), a surgical tool controller 1107 (e), a surgical tool 1122 (f), a shaft 1116 (g), a handle 1110 (h), a button 1114 (i), a transmitter 1210 (j), a motor 1212 (k), a communication circuit 1216 (1), a drive assembly 1204 (m), an identifier 1300 (n), and an identification signal reader 1302 (o), correspond respectively to the components of the manipulator system 500: the manipulator 10 (a), the controller 514 (b), the working unit 16 (c), the actuator block 30 (d), the connecting portion 15 (e), the end working portion 12 (f), the connecting shaft 48 (g), the grip handle 26 (h), the trigger lever 32 (i), the RFID 610 (j), the motors 41 to 42 (k), the transmitter-receiver 612 (1), the pulleys 50a to 50c (m), the image code 104 (n), and the camera 106 (o). The signs in the parentheses are shown in order to easily compare the corresponding components.

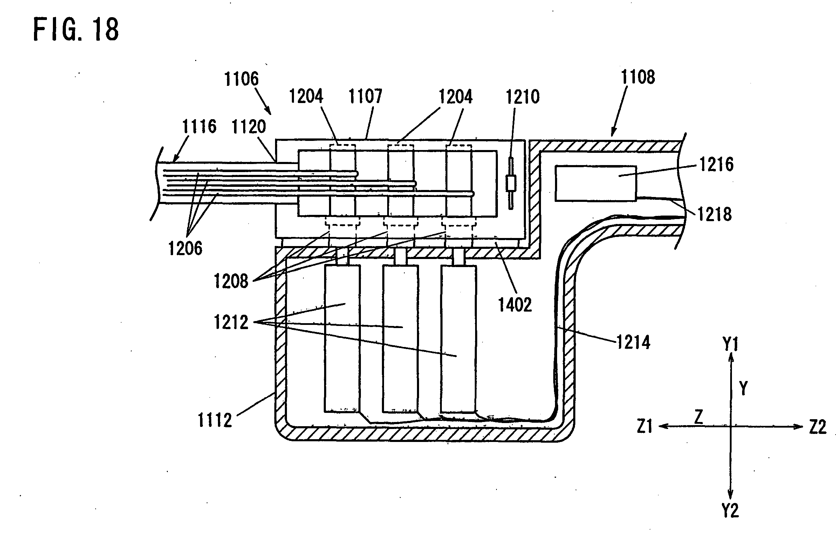

With reference to FIG. 18, a perspective view of a manipulator system 1100 is shown. The manipulator system 1100 may include a medical manipulator 1102 and a control unit 1104. The manipulator 1102 may include a surgical instrument (working unit) 1106 and a surgical tool control mechanism 1108. The surgical instrument 1106 may include a surgical tool controller 1107, a surgical tool 1122, and a shaft 1116. The shaft 1116 has a first end 1118 and a second end 1120 opposite to the first end 1118. The surgical tool 1122 mounts to the first end 1118 of the shaft 1116 using a variety of mechanisms as known to those skilled in the art both now and in the future. The surgical tool controller 1107 mounts to the second end 1120 of the shaft 1116 using a variety of mechanisms as known to those skilled in the art both now and in the future. As used in this disclosure, the term "mount" includes join, engage, unite, connect, associate, insert, hang, hold, affix, attach, fasten, bind, paste, secure, bolt, screw, rivet, solder, weld, and other like terms.

The surgical tool control mechanism 1108 may be mechanical, electro-mechanical, and/or electrical as known to those skilled in the art both now and in the future. The surgical tool control mechanism 1108 may include a handle 1110 and a surgical instrument control unit (actuator unit) 1112. A surgeon maneuvers and manipulates the handle 1110 to perform minimally invasive surgical procedures using the surgical tool 1122 as known to those skilled in the art both now and in the future. The handle 1110 may include a variety of control structures that may be rotated, depressed, toggled, etc. to indicate the desired movement of the surgical tool 1122. For example, the handle 1110 includes a button 1114 that the surgeon may depress to cause opening and closing of the surgical tool 1122. The surgical tool control mechanism 1108 is electrically connected to the control unit 1104 through cables within a harness 1115.

The control unit 1104 sends and receives electrical signals through the harness 1115 to/from the surgical tool control mechanism 1108 which controls movement of the surgical tool through a coupling with the surgical tool controller 1107. For example, control software receives electrical signals indicating movement of the button 1114 and transforms the movement to appropriate signals for an electro-mechanical system to effect movement of the surgical tool 1122. The electrical signals may be analog or digital. The control unit 1104 may further convert angle measurements received from transducers mounted in the handle 1110 to determine control commands that are transmitted to the surgical tool control mechanism 1108 which controls the surgical tool 1122.

With reference to FIG. 18, the surgical tool controller 1107 may include a plurality of drive assemblies 1204, a plurality of cables 1206, a plurality of connectors 1208, and a transmitter (ID holder) 1210. The transmitter 1210 may include a RFID. In general, the shaft 1116 includes an elongate tube through which the plurality of cables 1206 extend. The plurality of cables 1206 operably couple the surgical tool 1122 with the surgical tool controller 1107. The surgical tool 1122 may be of many different types including devices specifically designed for cutting, scraping, suturing, grasping, etc.

With further reference to FIG. 18, the surgical tool control mechanism 1108 may include a plurality of motors 1212, a plurality of control cables 1214, a communication circuit (ID recognizer) 1216, and a control wire 1218 mounted within the surgical instrument control unit 1112. The communication circuit 1216 may include a receiver. When the surgical instrument 1106 is mounted to the surgical tool control mechanism 1108, the transmitter 1210 communicates an identifier to the communication circuit 1216. The identifier identifies the type of the surgical tool 1122 so that the manipulator system 1100 operates as desired. The communication circuit 1216 receives the identifier and communicates the identifier to the control unit 1104 through the control wire 1218 contained within the harness 1115.

The control unit 1104 receives the identifier from the communication circuit 1216. The control unit 1104 interprets the movement considering the identified surgical tool type and provides control commands to the plurality of motors 1212 through the plurality of the control cables 1214 contained within the harness 1115. The surgical instrument 1106 is removable from the surgical tool control mechanism 1108 so that a variety of surgical tools may be easily and quickly interchanged. The plurality of motors 1212 releasably engage with the plurality of drive assemblies 1204 through the plurality of connectors 1208 to physically connect the surgical tool control mechanism 1108 to the surgical instrument 1106.

The plurality of drive assemblies 1204 include mechanical components that translate commands from the control unit 1104 into mechanical movement of the surgical tool 1122. For example, the plurality of motors 1212 create rotational torque that rotates the plurality of drive assemblies 1204. The rotation of the plurality of drive assemblies 1204 causes translational movement of the plurality of cables 1206 which causes movement of the surgical tool 1122. Because the surgical tool 1122 may comprise a variety of surgical devices for cutting, scraping, suturing, etc., the plurality of cables 1206 may effect different types of movement of the surgical tool 1122. Thus, the control unit 1104 recognizes the type of the surgical tool 1122 and generates appropriate control commands to the plurality of motors 1212 based on the type of the surgical tool 1122.

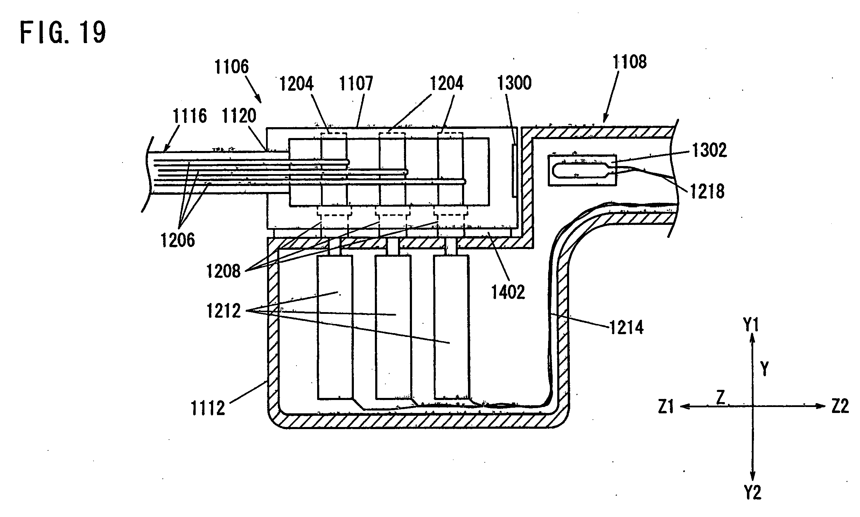

With reference to FIG. 19, the surgical tool controller 1107 may include the plurality of drive assemblies 1204, the plurality of cables 1206, the plurality of connectors 1208, and an identifier (ID holder) 1300. The identifier 1300 may be mounted to an exterior surface of the surgical instrument 1106. For example, the identifier 1300 is mounted to an exterior surface of the surgical tool controller 1107 adjacent to the surgical tool control mechanism 1108. The identifier 1300 may include a bar code as known to those skilled in the art both now and in the future. The surgical tool control mechanism 1108 may include the plurality of motors 1212, the plurality of control cables 1214, an identification reader (ID recognizer) 1302, and the control wire 1218 mounted within the surgical instrument control unit 1112. The identification reader 1302 may include a bar code reader that reads identification signals of the identifier 1300 using an infrared signal. When the instrument 1106 is mounted to the surgical tool control mechanism 1108, the identification reader 1302 reads identification signals of the identifier 1300 and communicates the signals of the identifier 1300 to the control unit 1104 through the control wire 1218 contained within the harness 1115. The control unit 1104 receives the identifier from the identification reader 1302. The control unit 1104 interprets the movement considering the identified surgical tool type and provides control commands to the plurality of motors 1212 through the plurality of control cables 1214 contained within the harness 1115 as discussed with reference to FIG. 19.

In the manipulator system 1100, the identification data can be contactless-transmitted between the surgical tool controller 1107 and the surgical tool control mechanism 1108, whereby the surgical tool controller 1107 has a structure with no electrical contacts and can be easily washed and sterilized.

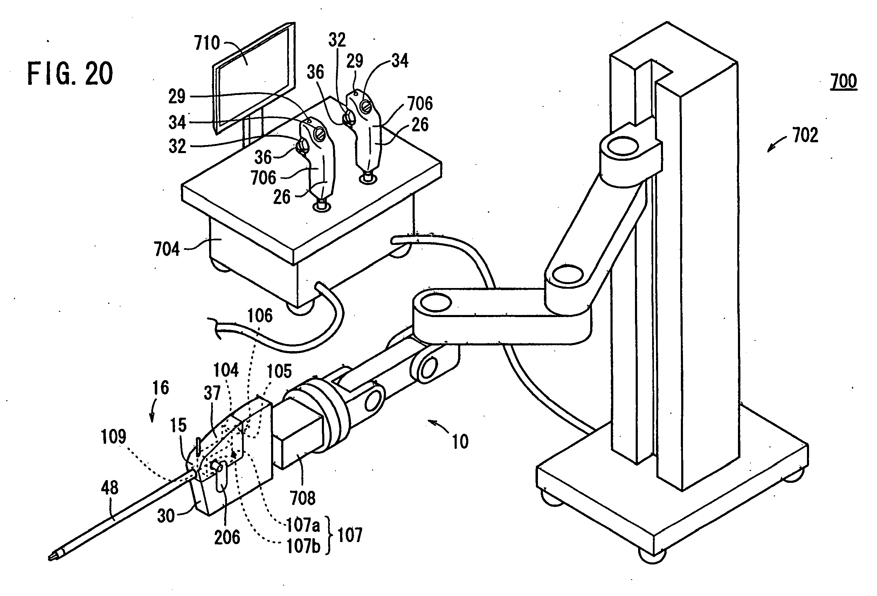

Although the above described working unit 16 is connected to the operating unit 14 handled by a human hand, the working unit can be used, for example, in a surgical robotic system 700 shown in FIG. 20.

The surgical robotic system 700 has a robotic arm 702 and a console 704, and the working unit 16 is connected to the distal end of the robotic arm 702. The robotic arm 702 has, at the distal end, the same mechanism as the above described actuator block 30, whereby the working unit 16 can be connected thereto and driven. A manipulator 10 in the system has the robotic arm 702 and the working unit 16. The robotic arm 702 may be of a stationary type, an autonomous mobile type, or the like, as long as it can move the working unit 16. The console 704 may be of a table type, a control panel type, or the like.

It is preferred that the robotic arm 702 has six or more independent joints (rotary shafts, slidable shafts, etc.), whereby the position and direction of the working unit 16 can be optionally controlled. The distal end 708 of the robotic arm 702 is integral with an actuator block 30.

The actuator block 30 has the camera 106 (or the transmitter-receiver 612), the white LEDs 105, and the working unit detecting means 107, and the connecting portion 15 of the working unit 16 has the image code 104 (or the RFID 610) and the detection piece 109.

The robotic arm 702 is driven under the control of the console 704, and may be driven by a program for automatic operation, by a joystick (a robotic operating unit) 706 disposed in the console 704, or by a combination thereof. The console 704 has a function of the above-described controller 514.

The console 704 has two joysticks 706 and a monitor 710, and the joysticks 706 are used as mechanisms provided by removing the actuator block 30 from the operating unit 14. Two robotic arms 702 can be independently controlled by the two joysticks 706 though not shown. The two joysticks 706 are positioned such that they can be easily handled by both hands. Information such as an endoscopic image is shown in the monitor 710.

The joysticks 706 can be moved upward, downward, rightward, or leftward, and can be twisted or tilted. The robotic arm 702 is moved in accordance with the motions. Each of the joysticks 706 may be a master arm. A communication means between the robotic arm 702 and the console 704 may be a wired or wireless means, a network means, or a combination thereof.

A manipulator (10) comprises an actuator block (30) and a working unit (16) attachable to and detachable from the actuator block (30). The actuator block (30) has a motor (40, 41, 42), the working unit (16) has a connecting shaft (48) and an end working portion (12) disposed at the distal end thereof, and the end working portion (12) is rotated in conjunction with the motor (40, 41, 42). The working unit (16) further has a two-dimensional code (104) holding an identification signal, and the actuator block (30) further has an infrared camera (106) for recognizing the identification signal in the code (104) and supplying it to a controller (514), is the camera (106) being not in contact with the code (104). The controller (514) controls the working unit (16) based on the supplied identification signal.

## Claims

1. A manipulator system comprising:
a manipulator (10); and
a controller (514) therefor,
said manipulator (10) comprising an actuator unit (30) containing an actuator (40, 41, 42), and a working unit (16) configured to be attached to and detached from said actuator unit (30) and containing a shaft (48, 1116) and an end working portion (12) disposed at a distal end of said shaft (48, 1116), said end working portion (12) configured for being rotated around an axis non-parallel to said shaft (48, 1116) and rotation of said end working portion being controlled by said actuator (40, 41, 42),
said working unit (16) comprising an ID holder (610, 1210, 1300) for holding an identification signal,
said actuator unit (30) comprising an ID recognizer (612, 1216, 1302) for recognizing said identification signal in said ID holder (610, 1210, 1300) and supplying said identification signal to said controller (514), said ID recognizer (612, 1216, 1302) being not in contact with said ID holder (610, 1210, 1300),
wherein said controller (514) is configured to control said working unit (16) based on said supplied identification signal,
wherein said ID holder (610, 1210, 1300) comprises an RFID which acts as a transmitter for transmitting said identification signal through a radio wave, and said ID recognizer (612, 1216, 1302) acts as a receiver for receiving said identification signal transmitted from said RFID,
**characterized in that**
said RFID is a 13.56-MHz-band, 2.45-GHz-band, or 5-GHz-band RFID, and said RFID is disposed such that, in a main lobe beam width of said RFID with a strongest radio wave directivity, a power pattern within -3 dB is directed to said ID recognizer (612, 1216, 1302).

2. A manipulator system according to claim 1, wherein said actuator unit (30) contains a data transmitter for transmitting a usage history data of said manipulator system through a radio wave, and said RFID is configured to receive said usage history data and to record it on a recording means.

3. A manipulator system according to claim 1, wherein said actuator unit (30) contains a working unit detecting means (107) for detecting the presence of said working unit (16), and
when said working unit (16) is attached to said actuator unit (30), based on a signal from said working unit detecting means (107), said controller (514) is configured to control said ID recognizer (612, 1216, 1302) to obtain said identification signal of said ID holder (610, 1210, 1300).

4. A manipulator system according to claim 3, wherein said working unit detecting means (107) comprises a phototransmitter and a photoreceiver facing each other, and a part of said working unit (16) is inserted between said phototransmitter and said photoreceiver so as to block a light from said phototransmitter, thereby detecting attachment of said working unit (16).

5. A manipulator system according to claim 1, wherein said controller (514) further comprises a usage history management means (602),
said actuator unit (30) contains a working unit detecting means (107) for detecting the presence of said working unit (16),
when said working unit (16) is attached to said actuator unit (30), based on a signal from said working unit detecting means (107), said controller (514) is configured to control said ID recognizer (612, 1216, 1302) to obtain said identification signal of said ID holder (610, 1210, 1300), and to receive a usage history data corresponding to said identification signal from said usage history management means (602), and after said working unit (16) is detached from said actuator unit (30), said usage history data is updated and transmitted to said usage history management means (602) by said controller (514) and is recorded on a recording means by said usage history management means (602), depending on said identification signal.

6. A manipulator system according to claim 1, wherein said manipulator system further comprises a usage history management means (602) configured to communicate with a plurality of controllers (514),
said actuator unit (30) contains a working unit detecting means (107) for detecting the presence of said working unit (16),
when said working unit (16) is attached to said actuator unit (30), based on a signal from said working unit detecting means (107), said controller (514) is configured to control said ID recognizer (612, 1216, 1302) to obtain said identification signal of said ID holder (610, 1210, 1300), and to receive a usage history data corresponding to said identification signal from said usage history management means (602), and after said working unit (16) is detached from said actuator unit (30), said usage history data is updated and transmitted to said usage history management means (602) by said controller (514) and is recorded on a recording means by said usage history management means (602), depending on said identification signal.

7. A manipulator system according to claim 6, wherein said usage history management means (602) is disposed in one or all of said controllers (514).

8. A manipulator system according to claim 1, wherein said end working portion (12) is connected to a connecting portion (15) by said shaft (48, 1116) in said working unit (16), said connecting portion (15) being connected to said actuator unit (30), and said ID holder (610, 1210, 1300) is provided in said connecting portion (15).

## Patentansprüche

1. Manipulatorsystem, mit:
einem Manipulator (10); und
einer Steuerung (514) für diesen,
wobei der Manipulator (10) eine Betätigungseinheit (30) mit einem Betätigungsglied (40, 41, 42) und eine Arbeitseinheit (16) aufweist, die konfiguriert ist, um an der Betätigungseinheit (30) angebracht und von dieser entfernt zu werden, und eine Welle (48, 1116) und einen Endarbeitsabschnitt (12) enthält, der an einem entfernten Ende der Welle (48, 1116) angeordnet ist, wobei der Endarbeitsabschnitt (12) dazu konfiguriert ist, um eine Achse, die zu der Welle (48, 1116) nicht parallel ist, gedreht zu werden, und die Drehung des Endarbeitungsabschnitts durch das Betätigungsglied (40, 41, 42) gesteuert wird,
wobei die Arbeitseinheit (16) eine ID-Halterung (610, 1210, 1300) zum Halten eines Identifikationssignals aufweist,
wobei die Betätigungseinheit (30) eine ID-Erkennungseinheit (612, 1216, 1302) zum Erkennen des Identifikationssignals in der ID-Halterung (610, 1210, 1300) und Zuführen des Identifikationssignals an die Steuerung (514) aufweist, wobei die ID-Erkennungseinheit (612, 1216, 1302) nicht mit der ID-Halterung (610, 1210, 1300) in Kontakt ist,
wobei die Steuerung (514) konfiguriert ist, um die Arbeitseinheit (16) basierend auf dem zugeführten Identifikationssignal zu steuern,
wobei die ID-Halterung (610, 1210, 1300) ein RFID umfasst, der als ein Sender zum Senden des Identifikationssignals über eine Funkwelle dient, und die ID-Erkennungseinheit (612, 1216, 1302) als ein Empfänger zum Empfangen des von dem RFID übertragenden Identifikationssignals dient,
**dadurch gekennzeichnet, dass**
das RFID ein 13,56-MHz Band, ein 2,45-GHz Band oder ein 5-GHz Band RFID ist, und das RFID derart angeordnet ist, das in einer Hauptkeulenstrahlbreite des RFID mit einer stärksten Funkwellenrichtcharakteristik ein Leistungsmuster innerhalb -3 dB auf die ID-Erkennungseinheit (612, 1216, 1302) gerichtet ist.

2. Manipulatorsystem gemäß Anspruch 1, wobei die Betätigungseinheit (30) einen Datensender zum Senden von Nutzungsverlaufsdaten des Manipulatorsystems über eine Funkwelle enthält und das RFID konfiguriert ist, um die Nutzungsverlaufsdaten zu empfangen und diese auf einer Aufzeichnungseinrichtung aufzuzeichnen.

3. Manipulatorsystem gemäß Anspruch 1, wobei die Betätigungseinheit (30) eine Arbeitseinheitserfassungseinrichtung (107) zum Erfassen des Vorhandenseins einer Arbeitseinheit (16) enthält, und
wenn die Arbeitseinheit (16) an der Betätigungseinheit (30) angebracht ist, basierend auf einem Signal von der Arbeitseinheiterfassungseinrichtung (107), die Steuerung (514) konfiguriert ist, die ID-Erkennungseinheit (612, 1216, 1302) zu steuern, um das Identifikationssignals der ID-Halterung (610, 1210, 1300) zu erhalten.

4. Manipulatorsystem gemäß Anspruch 3, wobei die Arbeitseinheitserfassungseinrichtung (107) einen Fotosender und einen Fotoempfänger umfasst, die einander gegenüberstehen, und ein Teil der Arbeitseinheit (16) zwischen dem Fotosender und dem Fotoempfänger eingesetzt wird, um Licht von dem Fotosender zu blockieren, um dadurch ein Anbringen der Arbeitseinheit (16) zu erfassen.

5. Manipulatorsystem gemäß Anspruch 1, wobei
die Steuerung (514) weiterhin eine Nutzungsverlaufsverwaltungseinrichtung (602) aufweist,
die Betätigungseinheit (30) eine Arbeitseinheitserfassungseinrichtung (107) zum Erfassen des Vorhandenseins der Arbeitseinheit (16) enthält,
die Steuerung (514), wenn die Arbeitseinheit (15) an der Betätigungseinheit (30) angebracht ist, basierend auf einem Signal der Arbeitseinheitserfassungseinrichtung (107), konfiguriert ist, um die ID-Erkennungseinheit (612, 1216, 1302) zu steuern, um das Identifikationssignal der ID-Halterung (610, 1210, 1300) zu erhalten und Nutzungsverlaufsdaten entsprechend dem Identifikationssignal von der Nutzungsverlaufsverwaltungseinrichtung (602) zu empfangen, und, nachdem die Arbeitseinheit (16) von der Betätigungseinheit (30) entfernt ist, die Nutzungsverlaufsdaten in Abhängigkeit des Identifikationssignals aktualisiert und an die Nutzungsverlaufsverwaltungseinrichtung (602) durch die Steuerung (514) übertragen werden und auf einer Aufzeichnungseinrichtung durch die Nutzungsverlaufsverwaltungseinrichtung (602) aufgezeichnet werden.

6. Manipulatorsystem gemäß Anspruch 1, wobei das Manipulatorsystem weiterhin eine Nutzungsverlaufsverwaltungseinrichtung (602) aufweist, die konfiguriert ist, um mit einer Vielzahl von Steuerungen (514) zu kommunizieren,
wobei die Betätigungseinheit (30) eine Arbeitseinheitserfassungseinrichtung (107) zum Erfassen des Vorhandenseins der Arbeitseinheit (16) enthält,
wobei, wenn die Arbeitseinheit (16) an der Betätigungseinheit (30) angebracht ist, basierend auf einem Signal der Arbeitseinheitserfassungseinrichtung (107), die Steuerung (514) konfiguriert ist, um die ID-Erkennungseinheit (612, 1215, 1302) zu steuern, um das Identifikationssignal der ID-Halterung (610, 1210, 1300) zu erhalten, und Nutzungsverlaufsdaten entsprechend dem Identifikationssignal von der Nutzungsverlaufsverwaltungseinrichtung (602) zu empfangen, und wobei, nachdem die Arbeitseinheit (16) von der Betätigungseinheit (30) entfernt ist, die Nutzungsverlaufsdaten in Abhängigkeit des Identifikationssignals aktualisiert und durch die Steuerung (514) an die Nutzungsverlaufsverwaltungseinrichtung (602) übertragen werden und auf einer Aufzeichnungseinrichtung durch die Nutzungsverlaufsverwaltungseinrichtung (602) aufgezeichnet werden.

7. Manipulatorsystem gemäß Anspruch 6, wobei die Nutzungsverlaufsverwaltungseinrichtung (602) in einer oder allen der Steuerungen (514) angeordnet ist.

8. Manipulatorsystem gemäß Anspruch 1, wobei der Endarbeitsabschnitt (12) mit einem Verbindungsabschnitt (15) durch die Welle (48, 1116) in der Arbeitseinheit (16) verbunden ist, wobei der Verbindungsabschnitt (15) mit der Betätigungseinheit (30) verbunden ist und die ID-Halterung (610, 1210, 1300) in dem Verbindungsabschnitt (15) bereitgestellt ist.

## Revendications

1. Système manipulateur comprenant:
un manipulateur (10); et
une unité de commande (514) correspondante,
ledit manipulateur (10) comprenant une unité (30) d'actionneur contenant un actionneur (40, 41, 42), et une unité (16) de travail configurée pour être attachée à ladite unité (30) d'actionneur et d'en être détachée et contenant un arbre (48, 1116) et une partie (12) de travail d'extrémité disposée au niveau d'une extrémité distale dudit arbre (48, 1116), ladite partie (12) de travail d'extrémité configurée pour être mise en rotation autour d'un axe qui n'est pas parallèle audit arbre (48, 1116) et la rotation de ladite partie de travail d'extrémité étant commandée par ledit actionneur (40, 41, 42),
ladite unité (16) de travail comprenant un support (610, 1210, 1300) d'ID pour maintenir un signal d'identification,
ladite unité (30) d'actionneur comprenant un dispositif de reconnaissance (612, 1216, 1302) d'ID pour reconnaitre ledit signal d'identification dans ledit support (610, 1210, 1300) d'ID et fournir ledit signal d'identification à ladite unité de commande (514), ledit dispositif de reconnaissance (612, 1216, 1302) d'ID n'étant pas en contact avec ledit support (610, 1210, 1300) d'ID,
où ladite unité de commande (514) est configurée pour commander ladite unité (16) de travail sur la base dudit signal d'identification fourni,
où ledit support (610, 1210, 1300) d'ID comprend une RFID qui agit en tant qu'émetteur pour émettre ledit signal d'identification à travers une onde radio, ledit dispositif de reconnaissance (612, 1216, 1302) d'ID agit en tant que récepteur pour recevoir ledit signal d'identification émis de ladite RFID,
**caractérisé en ce que**
ladite RFID est une RFID d'une bande de 13,56 MHz, d'une bande de 2,45 GHz, ou d'une bande de 5 GHz, et ladite RFID est disposée de sorte que, dans une largeur de faisceau du lobe principal de ladite RFID avec la plus forte directivité d'onde radio, un modèle de puissance à -3 dB est orienté vers ledit dispositif de reconnaissance (612, 1216, 1302) d'ID.

2. Système manipulateur selon la revendication 1, dans lequel ladite unité (30) d'actionneur contient un émetteur de données pour émettre des données d'historique d'utilisation dudit système manipulateur à travers une onde radio, et ladite RFID est configurée pour recevoir lesdites données d'historique d'utilisation et les enregistrer sur un moyen d'enregistrement.

3. Système manipulateur selon la revendication 1, dans lequel ladite unité (30) d'actionneur contient un moyen (107) de détection d'unité de travail pour détecter la présence de ladite unité (16) de travail, et
lorsque ladite unité (16) de travail est attachée à ladite unité (30) d'actionneur, sur la base d'un signal provenant dudit moyen (107) de détection d'unité de travail, ladite unité de commande (514) est configurée pour commander ledit dispositif de reconnaissance (612, 1216, 1302) d'ID afin d'obtenir ledit signal d'identification dudit support (610, 1210, 1300) d'ID.

4. Système manipulateur selon la revendication 3, dans lequel ledit moyen (107) de détection d'unité de travail comprend un photoémetteur et un photorécepteur se faisant face, et une partie de ladite unité (16) de travail est insérée entre ledit photoémetteur et ledit photorécepteur de sorte à bloquer une lumière dudit photoémetteur, détectant ainsi un attachement de ladite unité (16) de travail.

5. Système manipulateur selon la revendication 1, dans lequel ladite unité de commande (514) comprend en outre un moyen (602) de gestion d'historique d'utilisation,
ladite unité (30) d'actionneur contient un moyen (107) de détection d'unité de travail pour détecter la présence de ladite unité (16) de travail,
lorsque ladite unité (16) de travail est attachée à ladite unité (30) d'actionneur, sur la base d'un signal provenant dudit moyen (107) de détection d'unité de travail, ladite unité de commande (514) est configurée pour commander ledit dispositif de reconnaissance (612, 1216, 1302) d'ID pour obtenir ledit signal d'identification dudit support (610, 1210, 1300) d'ID, et pour recevoir des données d'historique d'utilisation correspondant audit signal d'identification provenant dudit moyen (602) de gestion d'historique d'utilisation, et après que ladite unité (16) de travail est détachée de ladite unité (30) d'actionneur, lesdites données d'historique d'utilisation sont mises à jour et transmises audit moyen (602) de gestion d'historique d'utilisation par ladite unité de commande (514) et sont enregistrées sur un moyen d'enregistrement par ledit moyen (602) de gestion d'historique d'utilisation, en fonction dudit signal d'identification.

6. Système manipulateur selon la revendication 1, dans lequel ledit système manipulateur comprend en outre un moyen (602) de gestion d'historique d'utilisation configuré pour communiquer avec une pluralité d'unités de commande (514),
ladite unité (30) d'actionneur contient un moyen (107) de détection d'unité de travail pour détecter la présence de ladite unité (16) de travail,
lorsque ladite unité (16) de travail est attachée à ladite unité (30) d'actionneur, sur la base d'un signal provenant dudit moyen (107) de détection d'unité de travail, ladite unité de commande (514) est configurée pour commander ledit dispositif de reconnaissance (612, 1216, 1302) d'ID afin d'obtenir ledit signal d'identification dudit support (610, 1210, 1300) d'ID, et recevoir des données d'historique d'utilisation correspondant audit signal d'identification provenant dudit moyen (602) de gestion d'historique d'utilisation, et après le détachement de ladite unité (16) de travail de ladite unité (30) d'actionneur, lesdites données d'historique d'utilisation sont mises à jour et transmises audit moyen (602) de gestion d'historique d'utilisation par ladite unité de commande (514) et sont enregistrées sur un moyen d'enregistrement par ledit moyen (602) de gestion d'historique d'utilisation, en fonction dudit signal d'identification.

7. Système manipulateur selon la revendication 6, dans lequel ledit moyen (602) de gestion d'historique d'utilisation est disposé dans l'une ou dans l'ensemble desdites unités de commande (514).

8. Système manipulateur selon la revendication 1, dans lequel ladite partie (12) de travail d'extrémité est reliée à une partie (15) de raccordement par ledit arbre (48, 1116) dans ladite unité (16) de travail, ladite partie (15) de raccordement étant reliée à ladite unité (30) d'actionneur, et ledit support (610, 1210, 1300) d'ID est pourvu dans ladite partie (15) de raccordement.
